# EUROPEAN PATENT APPLICATION

(11) **EP 4 574 037 A1**
(43) Date of publication of application: **25.06.2025**
(21) Application number: 24221906.1
(22) Date of filing: 19.12.2024
(51) Int. Cl.: A61B 5/145, A61B 5/1473, A61B 5/00

(54) **APPLICATOR AND APPLICATOR ASSEMBLY**

(30) Priority: 21.12.2023 KR 20230188467
(71) Applicant: i-Sens, Inc., Seoul 06646 (KR)
(72) Inventor: CHO, Hee Kyung, Seoul (KR); CHOI, Hyun Ho, Seoul (KR); PARK, Kyeongseok, Seoul (KR)
(74) Representative: HGF

(57) **Abstract**

An applicator according to an aspect of the present invention includes an insertion unit assembly which includes a needle and configured to move the needle in each of a first direction and a second direction which is a direction opposite to the first direction, an applicator body in which the insertion unit assembly is disposed and of which at least a part is formed of a molded pulp, and an operation part which is coupled to the insertion unit assembly in the applicator body and allows the needle to move in the first direction, wherein the operation part includes a button part which is movable in a third direction perpendicular to the first direction and of which at least a part is exposed to the outside of the applicator body, and a stopper which extends from the button part, prevents the insertion unit assembly from moving in the first direction, and allows the insertion unit assembly to move in the first direction by movement of the button part in the third direction.

## Description

### [Technical Field]

The present invention relates to an applicator and an applicator assembly.

### [Background Art]

Recently, with the advancement of medical technology, various types of medical devices that can be used directly by non-medical users have been developed and sold. As an example of such medical devices, there is a blood glucose meter that the user uses directly to monitor his or her own biometric data, such as a blood glucose level (blood glucose level).

Blood glucose meters are blood-collection type blood glucose meters and continuous glucose monitoring systems (CGMSs) (or continuous blood glucose meters).

Since a blood-collection type blood glucose meter requires a blood-collecting behavior of a user, it may be difficult to immediately monitor a hypoglycemic state.

A CGMS may increase the inconvenience to the user when used but is increasingly being used in that it can relatively immediately monitor biometric data.

Meanwhile, most applicators of the CGMS are discarded after one use, which can increase environmental pollution.

### [Related Art Document]

### [Patent Document]

(Patent Document 0001) Korean Laid-Open Patent No. 2021-0121666

### [Disclosure]

### [Technical Problem]

One of embodiments of the present invention is directed to providing an applicator and an applicator assembly, which are capable of increasing user convenience when a sensing device is attached to a body.

Another one of embodiments of the present invention is directed to providing an applicator and an applicator assembly, which are capable of attaching a sensing device to a body and then reducing environmental pollution when the applicator is discarded.

### [Technical Solution]

According to an aspect of the present invention, there is provided an applicator including an insertion unit assembly which includes a needle and configured to move the needle in each of a first direction and a second direction which is a direction opposite to the first direction, an applicator body in which the insertion unit assembly is disposed and of which at least a part is formed of a molded pulp, and an operation part which is coupled to the insertion unit assembly in the applicator body and allows the needle to move in the first direction, wherein the operation part includes: a button part which is movable in a third direction perpendicular to the first direction and of which at least a part is exposed to the outside of the applicator body, and a stopper which extends from the button part, prevents the insertion unit assembly from moving in the first direction, and allows the insertion unit assembly to move in the first direction by movement of the button part in the third direction.

According to another aspect of the present invention, there is provided an applicator assembly including a sensing device, and an applicator detachably coupled to the sensing device, wherein the applicator includes an applicator body which covers the sensing device and of which at least a part is formed of a molded pulp, an insertion unit assembly which is disposed in the applicator body and moves at least a part of the sensing device in a first direction; and an operation part which is disposed in the applicator body, coupled to the insertion unit assembly, and allows the at least a part of the sensing device to move in the first direction, and the operation part includes a button part which is movable in a third direction perpendicular to the first direction and of which at least a part is exposed to the outside of the applicator body; and a stopper which extends from the button part, prevents the insertion unit assembly from moving in the first direction, and allows the insertion unit assembly to move in the first direction by movement of the button part in the third direction.

### [Advantageous Effects]

According to embodiments of the present invention, it is possible to increase user convenience when a sensing device is attached to a body.

In addition, according to embodiments of the present invention, it is possible to reduce environmental pollution when an applicator is discarded.

### [Brief Description of the Drawings]

FIG. 1 is a schematic perspective view illustrating an applicator assembly according to one embodiment of the present invention.
FIG. 2 is a schematic view illustrating a state in which a sensing device of the applicator assembly is attached to a user's body according to one embodiment of the present invention.
FIG. 3 is a schematic view illustrating a state in which a sensor unit and a base unit of the sensing device are not coupled yet.
FIG. 4 is a schematic view illustrating a state in which the sensor unit and the base unit are coupled.
FIGS. 5 and 6 are schematic views illustrating a state in which the sensor unit is disassembled.
FIG. 7 is a schematic view illustrating a state in which a needle is coupled to the sensor unit.
FIG. 8 is a schematic view illustrating the base unit of the sensing device.
FIG. 9 is a schematic cross-sectional view along line I-I' in FIG. 1.
FIG. 10 is a schematic cross-sectional view along line II-II' in FIG. 1.
FIG. 11 is a schematic view illustrating a state in which an applicator assembly is disassembled according to one embodiment of the present invention.
FIG. 12 is a schematic view illustrating a state in which a base frame, a locking hook, and a moving tab among components of the applicator are disassembled.
FIG. 13 is a schematic top view illustrating a state in which the base frame and the locking hook of the applicator are coupled.
FIGS. 14A, 14B, and 14C are top views illustrating a state in which the base frame and the locking hook of the applicator assembly are coupled according to one embodiment of the present invention and sequentially show operation processes of the locking hook due to the movement of an operation part.
FIGS. 15 and 16 are schematic views illustrating a middle frame of the applicator.
FIG. 17 is a schematic view illustrating the operation part.
FIG. 18 is a schematic view illustrating a state in which an insertion unit assembly of the applicator is disassembled.
FIG. 19 is a schematic view illustrating a part of an intemal structure of the applicator assembly according to one embodiment of the present invention and is a schematic view illustrating a state in which the operation part is positioned at a deactivation position.
FIG. 20 is a schematic view illustrating a part of the intemal structure of the applicator assembly according to one embodiment of the present invention and is a schematic view illustrating a state in which the operation part is positioned at an activation position.
FIGS. 21A and 21B are schematic views illustrating a part of the internal structure of the applicator assembly according to one embodiment of the present invention and are schematic views illustrating the state in which the operation part is positioned at the deactivation position and the state in which the operation part is positioned at the activation position.
FIG. 22 is a schematic view illustrating an operation prevention part and a protective sheet applied to the present embodiment.
FIGS. 23 to 27 are schematic views illustrating a method of manipulating the applicator assembly according to one embodiment of the present invention, in which FIGS. 23, 24, and 26 are schematic cross-sectional views along line I-I' in FIG. 1, and FIGS. 25 and 27 are schematic cross-sectional views along line **II-II'** in FIG. 1.

### [Detailed Description of the Invention]

Terms used in the present application are only used to describe specific embodiments and are not intended to limit the present invention. The singular includes the plural unless the context clearly dictates otherwise. In the application, it should be understood that the terms "include" and "have" are intended to specify that a feature, a number, a step, an operation, a component, a part, or a combination thereof described in the specification is present, but do not preclude the possibility of the presence or addition of one or more other features, numbers, steps, operations, components, parts, or combinations thereof. In addition, throughout the specification, "on" means positioned above or under a target part and does not necessarily mean positioned above in a direction of gravity.

In addition, the term "coupling" is used as a concept that includes not only a case in which components are physically in direct contact with each other in a contact relationship between the components, but also a case in which another component is interposed between the components and the components are each in contact with another component.

In addition, the term "detaching" is used as a concept that means that two or more components coupled in a state in which no relative movement occurs therebetween move relatively with respect to each other by a user's manipulation or the like within a normal usage range.

Since the size and thickness of each component shown in the drawings are arbitrarily shown for convenience of description, the present invention is not necessarily limited to the shown ones.

Hereinafter, an applicator and an applicator assembly according to embodiments of the present invention will be described in detail with reference to the accompanying drawings, and in describing the embodiments of the present invention with reference to the accompanying drawings, the same or corresponding components are denoted as the same reference numeral, and overlapping description thereof will be omitted.

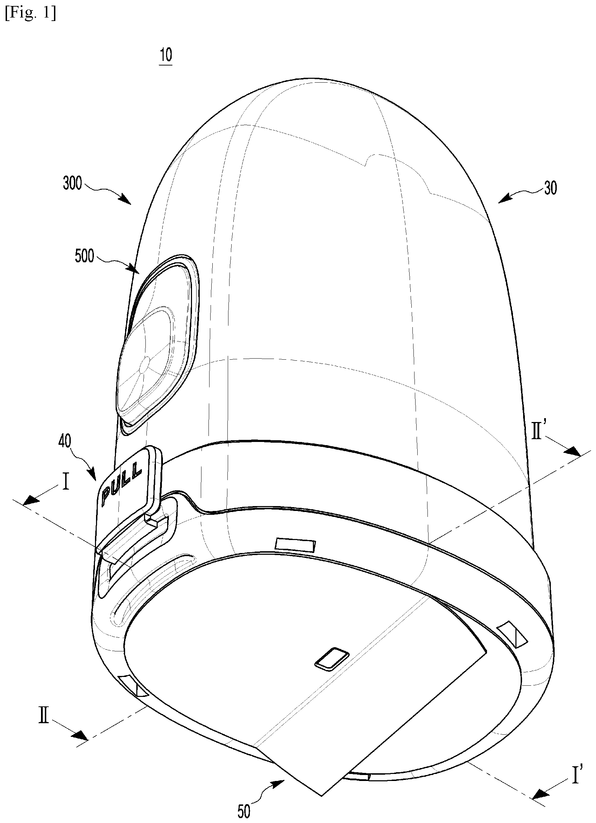

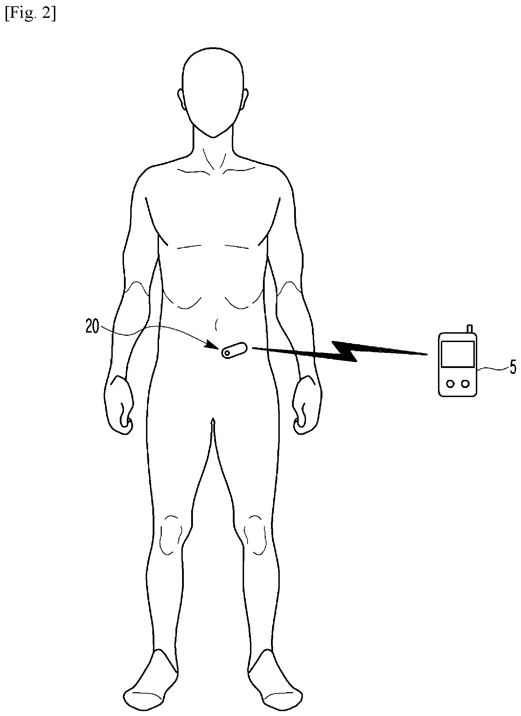

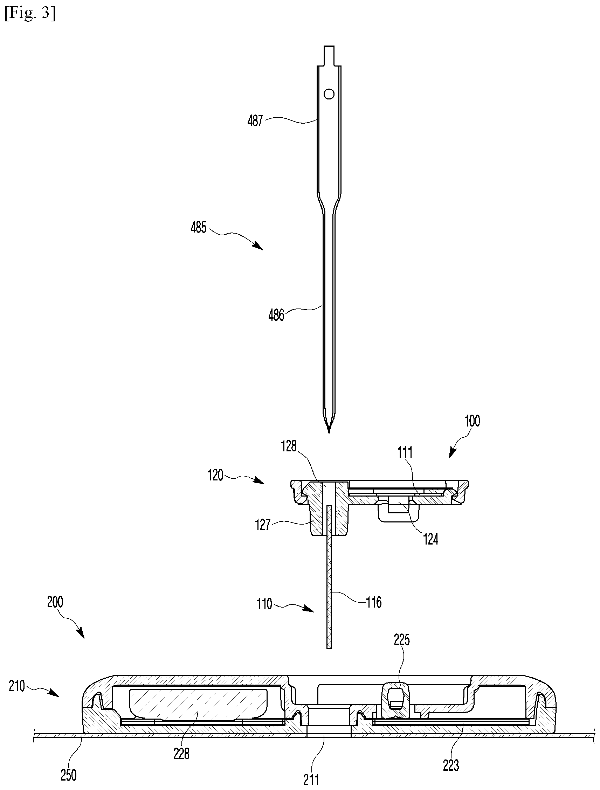

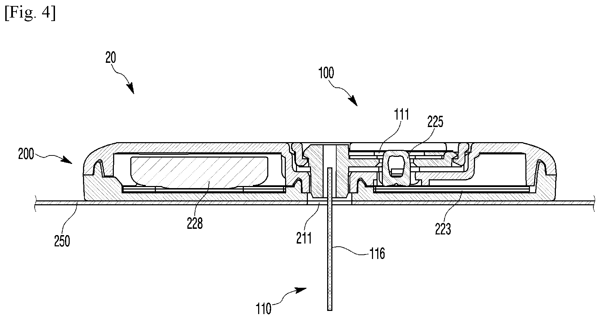

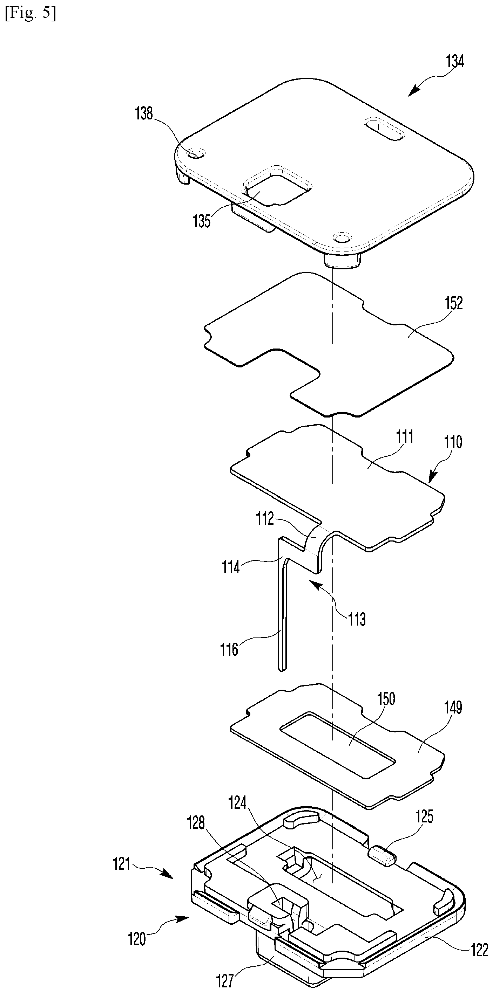

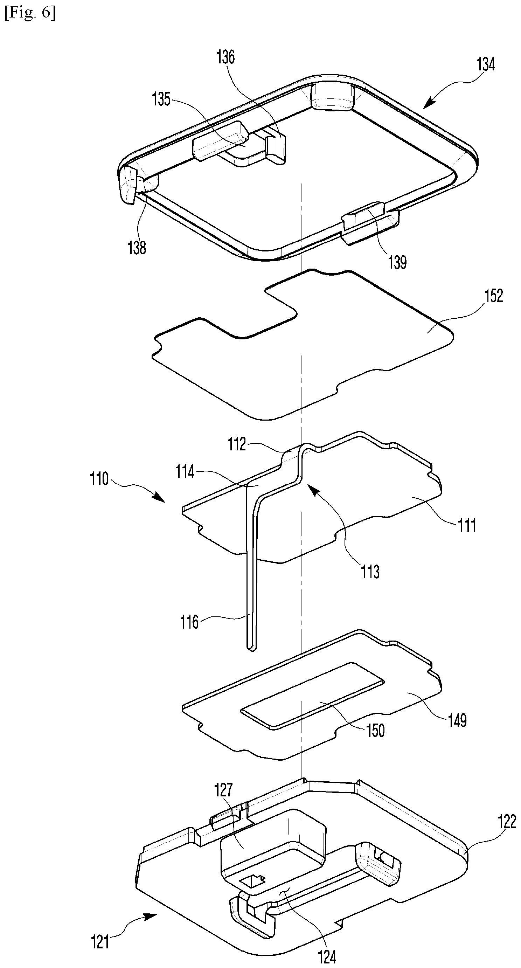

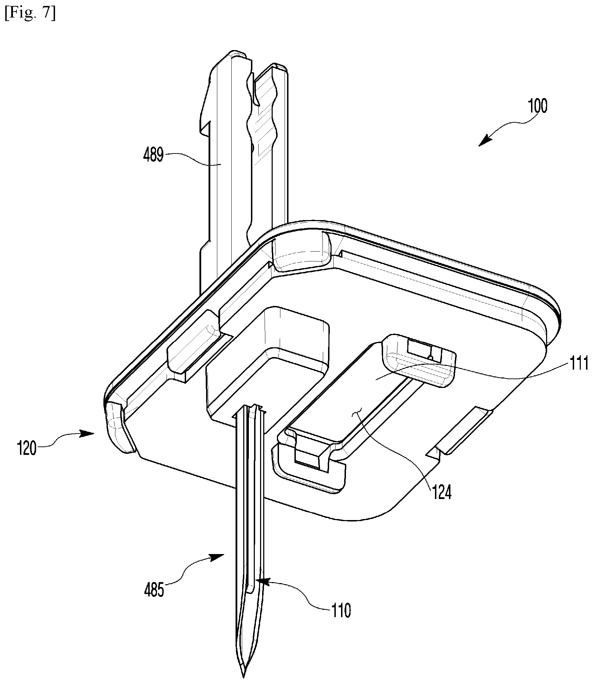

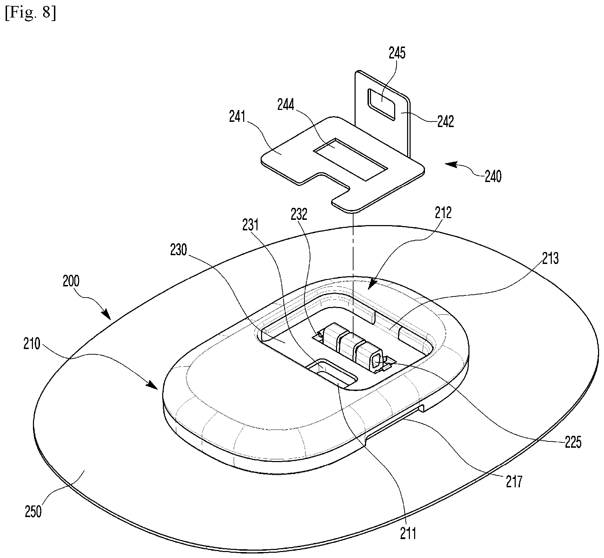

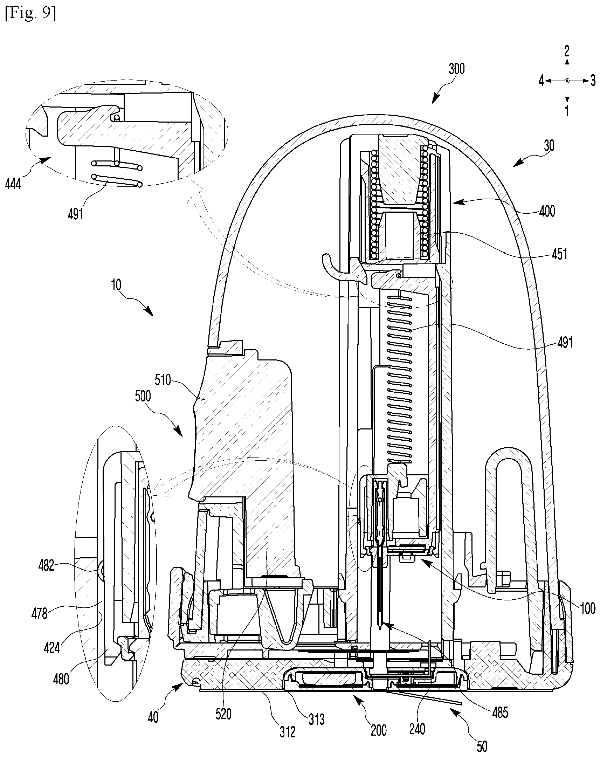

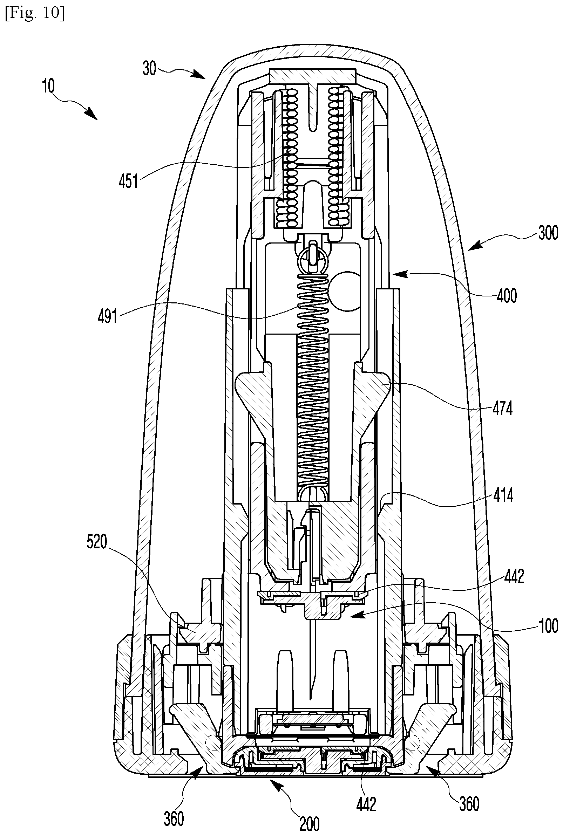

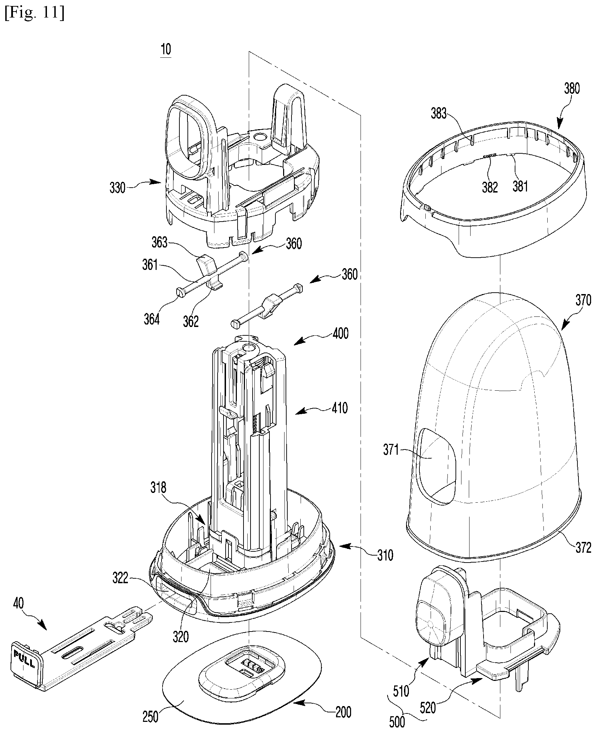

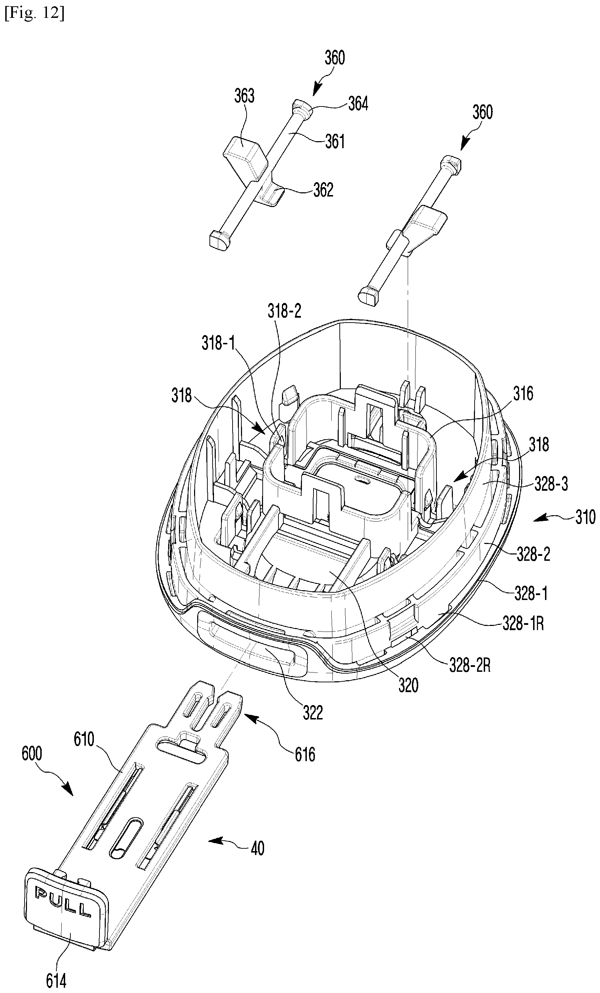

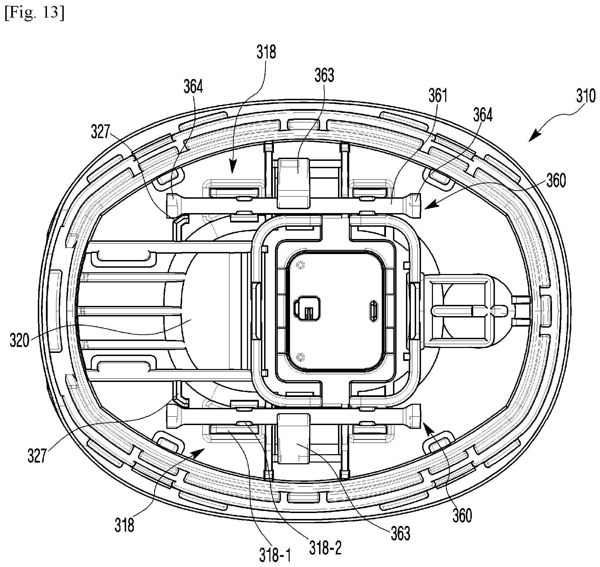

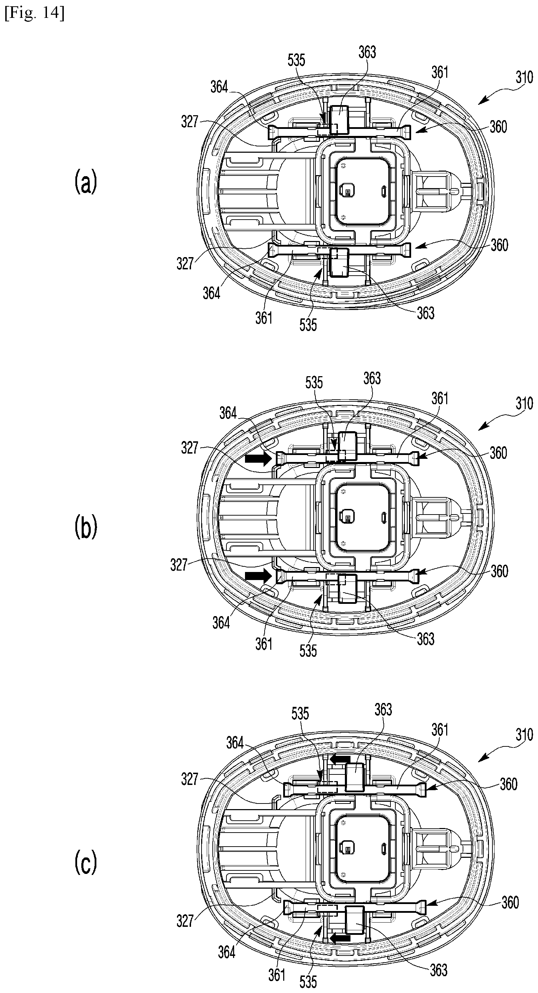

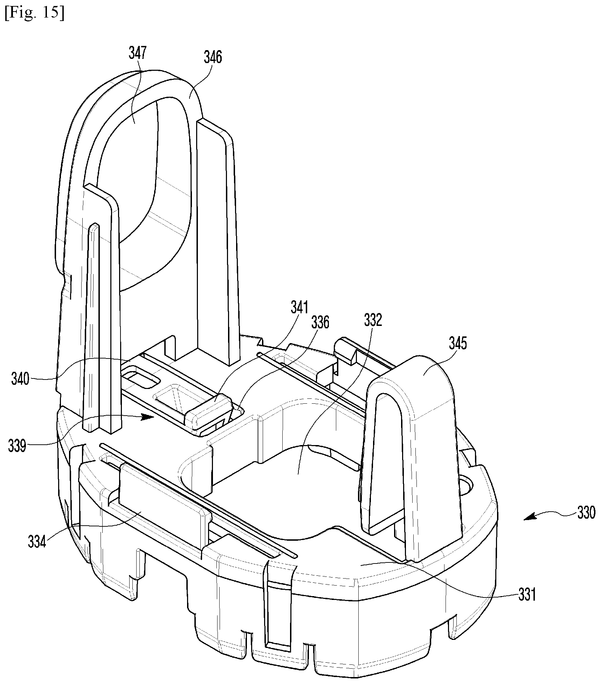

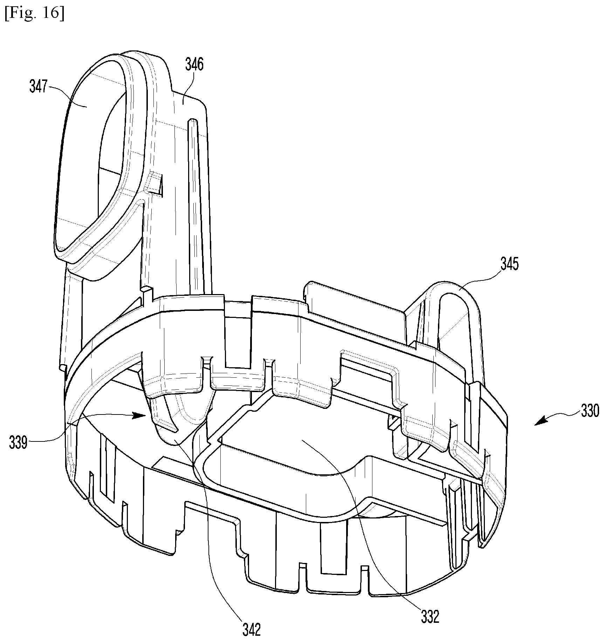

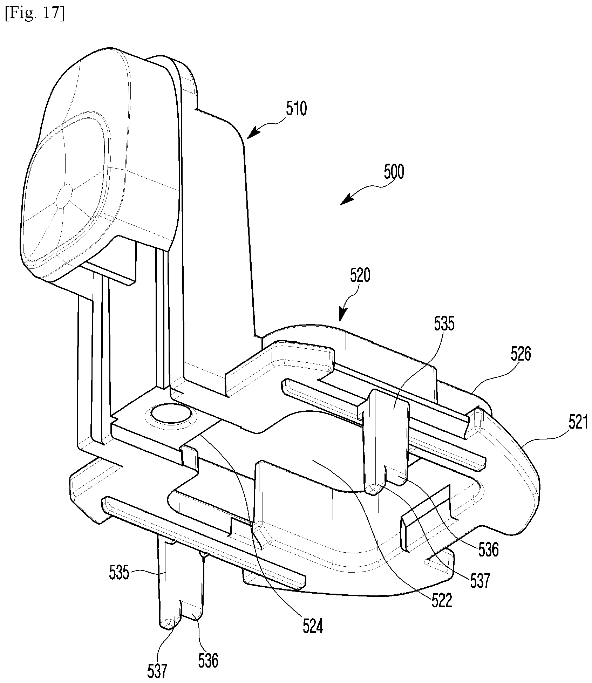

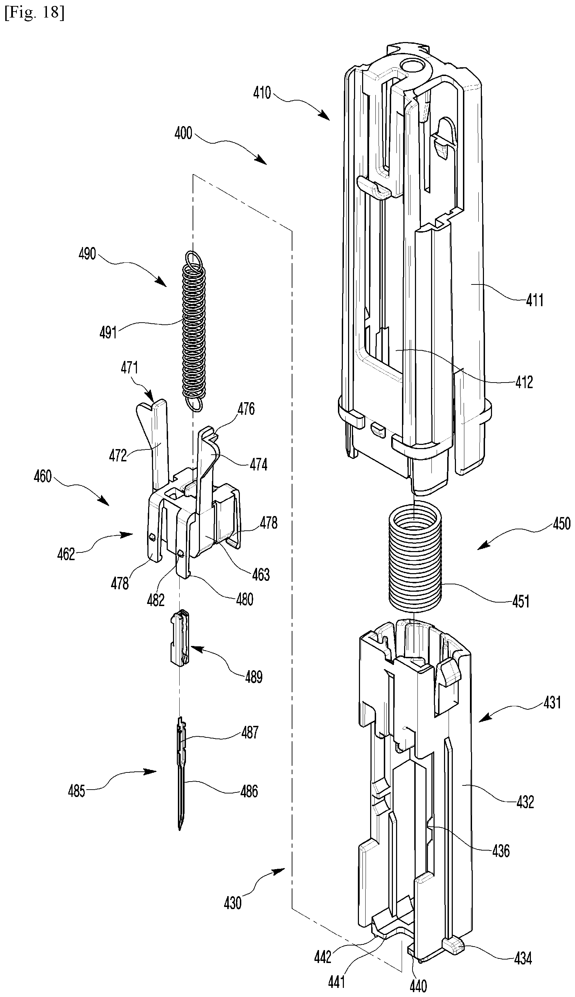

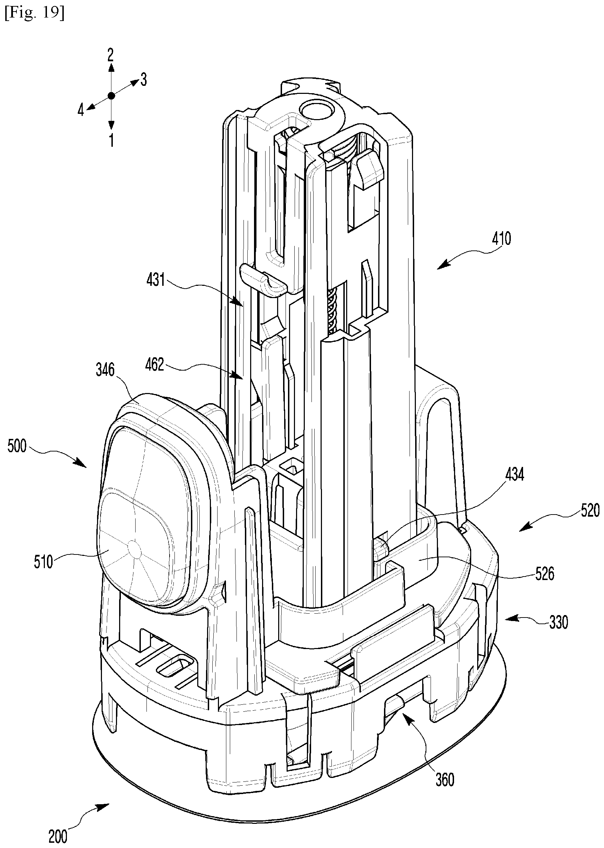

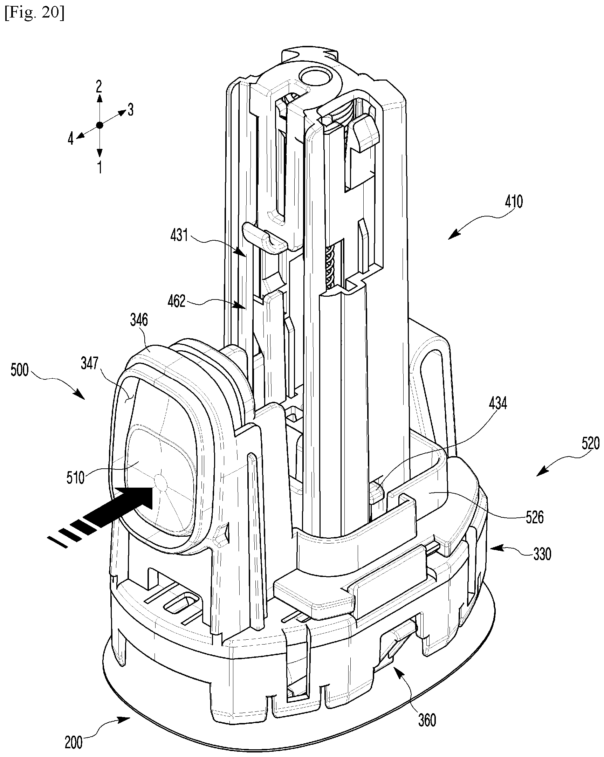

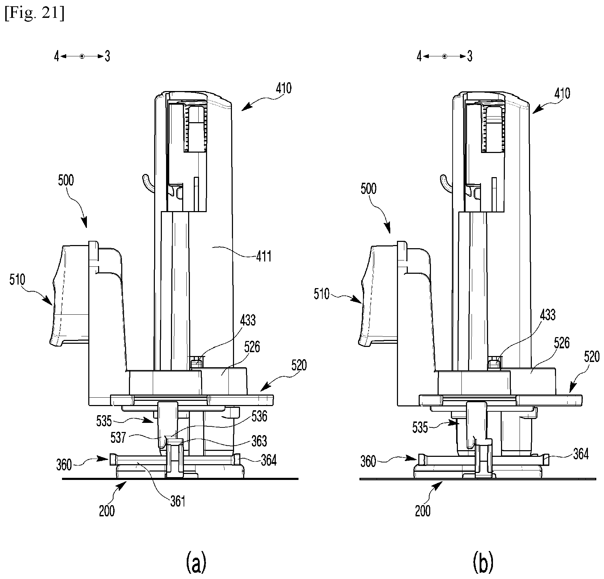

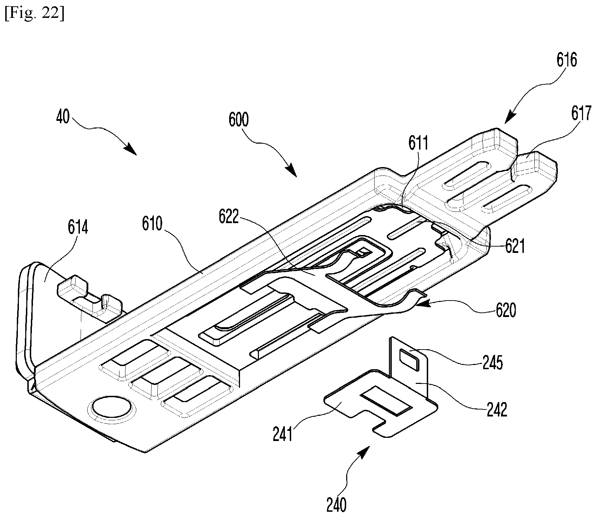

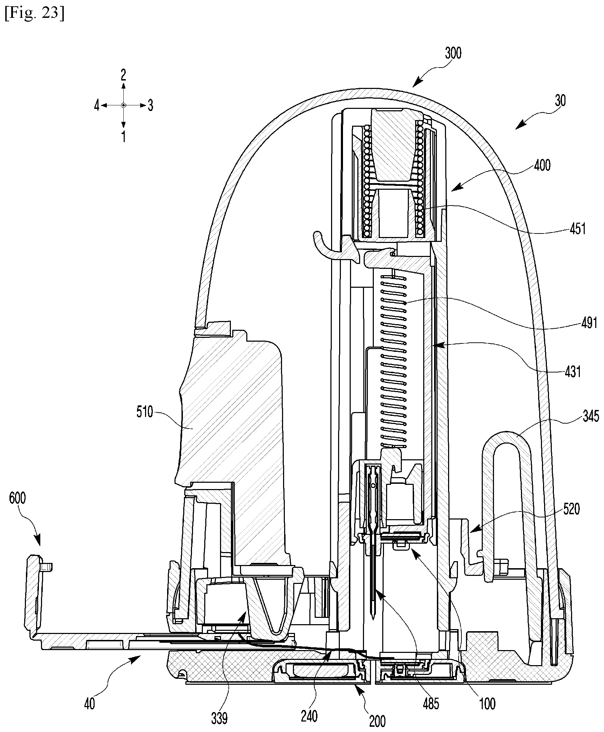

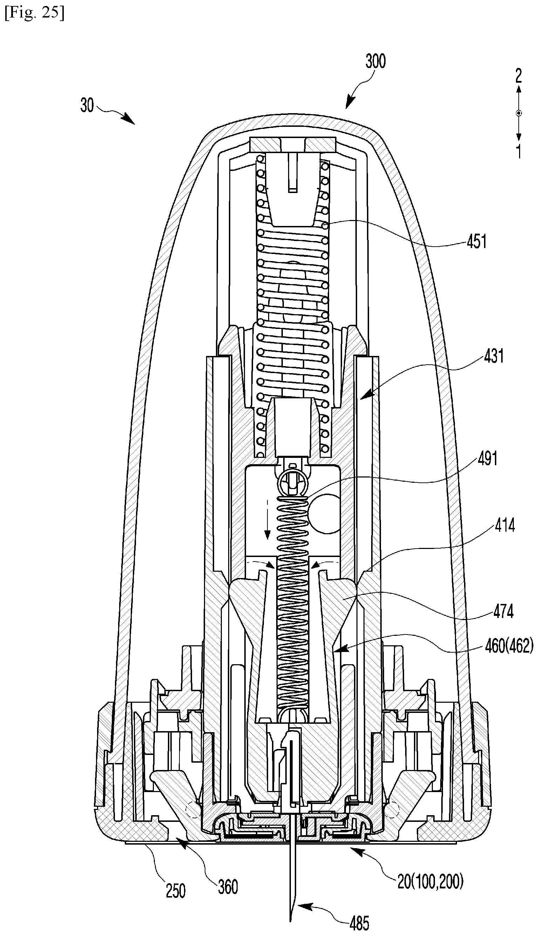

FIG. 1 is a schematic perspective view illustrating an applicator assembly according to one embodiment of the present invention. FIG. 2 is a schematic view illustrating a state in which a sensing device of the applicator assembly is attached to a user's body according to one embodiment of the present invention. FIG. 3 is a schematic view illustrating a state in which a sensor unit and a base unit of the sensing device are not coupled yet. FIG. 4 is a schematic view illustrating a state in which the sensor unit and the base unit are coupled. FIGS. 5 and 6 are schematic views illustrating a state in which the sensor unit is disassembled. FIG. 7 is a schematic view illustrating a state in which a needle is coupled to the sensor unit. FIG. 8 is a schematic view illustrating the base unit of the sensing device. FIG. 9 is a schematic cross-sectional view along line I-I' in FIG. 1. FIG. 10 is a schematic cross-sectional view along line II-II' in FIG. 1. FIG. 11 is a schematic view illustrating a state in which an applicator assembly is disassembled according to one embodiment of the present invention. FIG. 12 is a schematic view illustrating a state in which a base frame, a locking hook, and a moving tab among components of the applicator are disassembled. FIG. 13 is a schematic top view illustrating a state in which the base frame and the locking hook of the applicator are coupled. FIGS. 14A, 14B, and 14C are top views illustrating a state in which the base frame and the locking hook of the applicator assembly are coupled according to one embodiment of the present invention and sequentially show operation processes of the locking hook due to the movement of an operation part. FIGS. 15 and 16 are schematic views illustrating a middle frame of the applicator. FIG. 17 is a schematic view illustrating the operation part. FIG. 18 is a schematic view illustrating a state in which an insertion unit assembly of the applicator is disassembled. FIG. 19 is a schematic view illustrating a part of an intemal structure of the applicator assembly according to one embodiment of the present invention and is a schematic view illustrating a state in which the operation part is positioned at a deactivation position. FIG. 20 is a schematic view illustrating a part of the internal structure of the applicator assembly according to one embodiment of the present invention and is a schematic view illustrating a state in which the operation part is positioned at an activation position. FIGS. 21A and 21B are schematic views illustrating a part of the intemal structure of the applicator assembly according to one embodiment of the present invention and are schematic views illustrating the state in which the operation part is positioned at the deactivation position and the state in which the operation part is positioned at the activation position. FIG. 22 is a schematic view illustrating an operation prevention part and a protective sheet applied to the present embodiment. FIGS. 23 to 27 are schematic views illustrating a method of manipulating the applicator assembly according to one embodiment of the present invention, in which FIGS. 23, 24, and 26 are schematic cross-sectional views along line I-I' in FIG. 1, and FIGS. 25 and 27 are schematic cross-sectional views along line II-II' in FIG. 1.

Referring to FIGS. 1 to 27, an applicator assembly 10 according to one embodiment of the present invention includes a sensing device 20, an applicator 30, an operation prevention part 40, and a protective sheet 50.

The sensing device 20 may detect biometric data at each of different time points while attached to a user's skin and wirelessly transmit detected data to an external terminal 5. Here, the biometric data may be a blood glucose level, but the scope of the present invention is not limited thereto. Here, the different time points may be, for example, at least three or more time points with substantially the same cycle, but the scope of the present invention is not limited thereto. The external terminal 5 may be any of various devices capable of receiving detected data of the sensing device 20, such as a user's portable terminal, a dedicated medical device, a PC, a server, and the like.

The sensing device 20 includes a sensor unit 100 and a base unit 200. The sensing device 20 in a form in which the sensor unit 100 and the base unit 200 are spaced apart from each other within the applicator 30 to be described below may be provided to the user. As the user manipulates the applicator 30, the sensor unit 100 and the base unit 200 of the sensing device 20 may be coupled. The sensing device 20 may be detached from the applicator 30 and then attached to the user's body. In the present specification, the concept of the sensing device 20 includes both a state in which the sensor unit 100 and the base unit 200 are spaced apart from each other and a state in which the sensor unit 100 and the base unit 200 are coupled.

Meanwhile, in the present specification, it is assumed that the sensing device 20 includes the sensor unit 100 and the base unit 200 that are disposed to be spaced apart from each other in the applicator 30 and are coupled by the user's manipulation of the applicator 30. However, such description does not exclude other types of sensing devices from the scope of the present invention. As another non-limiting example, the sensor unit 100 and the base unit 200 may be disposed in a coupled form in the applicator 30 and moved in a first direction (see a direction illustrated in FIGS. 19, 20, and 23 to 27) by the user's manipulation of the applicator 30 while maintaining the coupled state to be attached to the user's skin and detached from the applicator 30 while maintaining the coupled state.

At least a part of the sensor unit 100 may be inserted into the user's skin to detect biometric data and generate a signal. The sensor unit 100 includes a sensor 110, a sensor unit housing 120, a sensor adhesive part 149, and an adhesive pad 152.

At least a part of the sensor 110 may be inserted into the user's skin to detect biometric data. The sensor 110 includes a sensor body 111, a connection portion 112, a middle portion 113, an extension 114, and an insertion portion 116.

The sensor body 111 may be disposed inside the sensor unit housing 120 to be described below. The connection portion 112 may extend from one side of the sensor body 111 to connect the sensor body 111 to the middle portion 113. The connection portion 112 may be formed so that the sensor body 111 and the middle portion 113 are disposed on different planes. The middle portion 113 may extend from one side of the connection portion 112 to connect the connection portion 112 to the insertion portion 116. The middle portion 113 may include the extension 114 protruding from the connection portion 112 to one side thereof. The insertion portion 116 may be connected to the extension 114 and inserted into the user's skin. The insertion portion 116 may be formed in a relatively thin and long probe shape to be smoothly inserted into the user's skin.

For example, the sensor body 111, the connection portion 112, the middle portion 113, and the insertion portion 116 may be manufactured in a flat shape, and then the connection portion 112 may be bent. Due to the bending of the connection portion 112, the middle portion 113 and the insertion portion 116 may be disposed on different planes from the sensor body 111, but the scope of the present invention is not limited thereto.

Meanwhile, FIGS. 5 and 6 illustrate that the middle portion 113 and the insertion portion 116 are disposed to be coplanar and the middle portion 113 is disposed vertically with the sensor body 111, but an angle between the middle portion 113 and the sensor body 111 or an angle between the insertion portion 116 and the sensor body 111 may be changed in various ways.

Meanwhile, conductive traces electrically connected to electrodes of the sensor body 111 may be disposed on the connection portion 112, the middle portion 113, and the insertion portion 116.

The sensor unit housing 120 accommodates at least a part of the sensor 110 therein. For example, the sensor body 111, the connection portion 112, and the middle portion 113 of the sensor 110 may be disposed inside the sensor unit housing 120, and at least a part of the insertion portion 116 of the sensor 110 may protrude from the sensor unit housing 120. The sensor unit housing 120 includes a housing base 121 and a housing cap 134.

The housing base 121 includes a base part 122 that supports the sensor body 111 and a boss 127 protruding from a surface of the base part 122. A through hole 124 may be formed in one side of the base part 122. An electrical contact part 225 to be described below may be inserted into the through hole 124. A housing base hole 128 may be formed in the boss 127. The insertion portion 116 and a needle 485 to be described below may be inserted into the housing base hole 128. A detent protrusion 125 may be formed at an edge of the base part 122. When the housing base 121 and a housing cap 134 to be described below are coupled, the detent protrusion 125 may be inserted into a detent groove 139 of the housing cap 134.

The housing cap 134 may be coupled to the housing base 121 to cover the sensor body 111. A housing cap hole 135 may be formed at one side of the housing cap 134. The housing cap hole 135 is connected to the housing base hole 128 of the housing base 121. Accordingly, the needle 485 may pass through the housing cap hole 135 and the housing base hole 128 and protrude outward from the sensor unit housing 120. The detent groove 139 may be formed in the housing cap 134. When the housing base 121 and the housing cap 134 are coupled, the detent protrusion 125 of the housing base 121 may be inserted into the detent groove 139. A sensor unit groove 138 may be formed in the housing cap 134. A push protrusion 442 of a shuttle 431 may be inserted into the sensor unit groove 138. The sensor unit groove 138 is formed to protrude from an inner surface of the housing cap 134 facing the housing base 121. A region in which the sensor unit groove 138 protrudes from the inner surface of the housing cap 134 may be inserted into a housing engagement portion of the housing base 121. A retention protrusion 136 may be formed on the inner surface of the housing cap 134 facing the housing base 121. The retention protrusion 136 may be disposed so that an end portion faces the middle portion 113 inserted into the housing base hole 128 to restrict the movement of the middle portion 113 of the sensor 110. Due to the retention protrusion 136, the insertion portion 116 of the sensor 110 may stably maintain a state of protruding a predetermined length from the sensor unit housing 120. In addition, due to the retention protrusion 136, the insertion portion 116 of the sensor 110 may maintain a state of being inserted into the user's skin to a predetermined depth without moving backward from the user's skin while inserted into the user's skin.

The sensor adhesive part 149 may be disposed between the housing base 121 and the sensor body 111 to attach the sensor body 111 to the housing base 121. The sensor adhesive part 149 may be formed in the form of a double-sided tape of which both surfaces have adhesive properties, but the scope of the present invention is not limited thereto. A sensor adhesive part opening 150 may be formed in a central portion of the sensor adhesive part 149. The electrical contact part 225 to be described below may be inserted into the sensor adhesive part opening 150.

The adhesive pad 152 may be disposed between the housing cap 134 and the sensor body 111 to attach the sensor body 111 to the housing cap 134. The adhesive pad 152 may be formed in the form of a double-sided tape of which both surfaces have adhesive properties, but the scope of the present invention is not limited thereto. An adhesive pad engagement portion may be formed on the adhesive pad 152. Through the adhesive pad engagement portion, the sensor unit groove 138 protruding to the inner surface of the housing cap 134 may be inserted into the housing engagement portion of the housing base 121.

The base unit 200 may be coupled to the sensor unit 100 and attached to the user's skin. The base unit 200 may be electrically connected to the sensor unit 100 to process the biometric data measured by the sensor unit 100 and transmit the processed biometric data to the external terminal 5. The base unit 200 includes a base unit housing 210, an electronic component, an adhesive layer 230, a protective sheet 240, and an adhesive part 250.

The base unit housing 210 may be coupled to the sensor unit housing 120. The base unit housing 210 may form an overall exterior of the base unit 200. An insertion hole 211 may be formed in the base unit housing 210. The insertion hole 211 may be formed in a form into which the boss 127 of the sensor unit 100 may be inserted so that at least a part of the insertion portion 116 of the sensor 110 may be inserted into the insertion hole 211. A mounting part 212, which has a region in which the sensor unit housing 120 is accommodated, may be formed in the base unit housing 210. The mounting part 212 includes a base unit recess 213. The sensor unit housing 120 may be coupled to the base unit recess 213. A housing groove 217 may be formed in the base unit housing 210. At least a part of a locking hook 360 of the applicator 30 to be described below may be inserted into the housing groove 217. The electrical contact part 225 may be formed in the base unit housing 210. The electrical contact part 225 may be in contact with the sensor body 111 after passing through the through hole 124 and the sensor adhesive part opening 150 of the sensor unit 100 when the sensor unit 100 is coupled to the base unit 200. The electrical contact part 225 may be formed in a form that may be elastically deformed for stable contact with the sensor body 111. The electrical contact part 225 may be electrically connected to a substrate 223 to be described below.

The electronic component is disposed inside the base unit housing 210. The electronic component may include the substrate 223, the electrical contact part 225 in contact with the sensor 110, a battery 228, and various chips. The chips may be disposed on the substrate 223. The chip may include a processor chip for processing signals and a communication chip for communicating with the external terminal 5.

As a modified example, the base unit 200 may exclude at least some of the electronic components. As an example, the base unit 200 may be composed of only a component for attaching the sensor unit 100 to the user's skin. In this case, a separate electronic unit may be detachably attached to the base unit 200. The electronic unit may include a processor chip for processing the biometric data detected by the sensor unit 100, and a communication chip for transmitting processed detected data to the external terminal 5. In addition, the electronic unit may be electrically connected to the sensor unit 100 after the sensor unit 100 is coupled to the base unit 200.

The adhesive layer 230 may be disposed on one surface of the base unit housing 210 to couple the sensor unit housing 120 with the base unit housing 210. The adhesive layer 230 may seal a gap between the sensor unit housing 120 and the base unit housing 210 to prevent moisture or foreign substances from being introduced into the electrical contact part 225. Adhesive layer holes 231 and 232 may be formed in the adhesive layer 230. Since the adhesive layer hole 231 is connected to the insertion hole 211, the needle 485 and the insertion portion 116 of the sensor 110 may pass through the adhesive layer hole 231. The electrical contact part 225 may pass through the adhesive layer hole 232. The adhesive layer 230 may be formed in the form of a double-sided tape of which both surfaces have adhesive properties.

The protective sheet 240 may cover the adhesive layer 230 to prevent the adhesive properties of the adhesive layer 230 from being reduced. Since the protective sheet 240 includes a releasable material, the protective sheet 240 can be easily removed from the adhesive layer 230 before the sensor unit 100 is coupled to the base unit 200 to expose the adhesive layer 230. The protective sheet 240 may include a protective portion 241 covering the adhesive layer 230, and a wing 242 extending from the protective portion 241. A protective sheet opening 244 may be formed in the protective portion 241. The protective sheet opening 244 may be connected to the adhesive layer hole 232 of the adhesive layer 230. A protective sheet hole 245 may be formed in the wing 242. Since the wing 242 protrudes from the base unit housing 210, the wing 242 and the protective sheet hole 245 may be coupled to a moving tab 600 of the applicator 30 to be described below.

The base unit 200 is coupled to the applicator 30 in a state in which the protective sheet 240 is attached to the adhesive layer 230. The protective sheet 240 may be detached from the adhesive layer 230 by the moving tab 600 to be described below before the sensor unit 100 is coupled to the base unit 200. The protective sheet 240 is detached from the adhesive layer 230 by the user manipulating the moving tab 600. Then, by the user manipulating the applicator 30 to be described below, the sensor unit 100 may be moved in the first direction (see the directions illustrated FIGS. 19, 20, and 23 to 27) and coupled to the base unit 200.

The adhesive part 250 may be disposed on one surface of the base unit housing 210 to attach the base unit housing 210 to the user's skin. An adhesive part opening through which the insertion portion 116 of the sensor 110 and the needle 485 may pass may be formed in a central portion of the adhesive part 250.

The applicator 30 forms an overall exterior of the applicator assembly 10 according to the present embodiment. The applicator 30 may cover at least a part of each of the sensor unit 100 and the base unit 200. The applicator 30 may couple the sensor unit 100 with the base unit 200 to form the sensing device 20. The applicator 30 may be detached from the sensing device 20 after the sensor unit 100 and the base unit 200 are coupled to form the sensing device 20. The applicator 30 may include an applicator body 300, an insertion unit assembly 400, and an operation part 500.

The applicator body 300 may form an overall exterior of the applicator 30. The applicator body 300 covers at least a part of each of the sensor unit 100 and the base unit 200 before the user manipulates the applicator 30. The applicator body 300 includes main bodies 310 and 330, the locking hook 360, a top case 370, and a top case restriction member 380.

The main bodies 310 and 330 accommodate at least a part of the insertion unit assembly 400 to be described below and support the insertion unit assembly 400. Hereinafter, for convenience of description, it is assumed that the main bodies 310 and 330 include the base frame 310 and the middle frame 330 that are individually formed and coupled. However, such description does not exclude the integrally formed main body from the scope of the present invention.

The base frame 310 may be in contact with the user's skin when the user uses the applicator assembly 10 according to the present embodiment. Specifically, in the above usage environment of the user, a bottom portion 312 of the base frame 310 may be in contact with the user's skin. A recess 313 may be formed in the bottom portion 312. At least a part of the base unit 200 may be accommodated in the recess 313 so that the base unit 200 may be detachably coupled to the base frame 310.

A base frame opening 314 may be formed in a central portion of the base frame 310. A fence part 316 may be disposed around the base frame opening 314. At least a part of the insertion unit assembly 400 to be described below may be inserted into the base frame opening 314, and the fence part 316 may support the insertion unit assembly 400. Accordingly, the base frame 310 may support the insertion unit assembly 400.

A Support 318 to which the locking hook 360 is coupled may be formed on the base frame 310. The support 318 may rotatably support the locking hook 360 to be described below. The support 318 may be formed at both sides of the base frame opening 314. The support 318 includes an elastic support 318-1 that is elastically deformable, and a support protrusion 318-2 protruding from a surface of the elastic support 318-1. The support protrusion 318-2 presses the locking hook 360 to prevent the locking hook 360 from being separated between the elastic support 318-1 and the fence part 316. In addition, an elastically deformable retention protrusion 327 may be formed on the base frame 310 to restrict the movement of the locking hook 360. The support 318 and the retention protrusion 327 will be described below.

A stage 320 may be formed inside the base frame 310. The stage 320 may support the moving tab 600 so that the moving tab 600 to be described below may linearly move stably. A doorway 322 may be formed in the base frame 310. The doorway 322 is formed at one side of an outer surface of the base frame 310. The moving tab 600 disposed in the stage 320 may be drawn out to the outside of the base frame 310 through the doorway 322. An applicator body engagement part 324 may be formed in the base frame 310. The applicator body engagement part 324 includes an engagement protrusion 325 formed inside the base frame 310 to be engaged with the moving tab 600.

External fences 328-1, 328-2, 328-3 are formed on the base frame 310. The external fences 328-1, 328-2, 328-3 include a first external fence 328-1 having a relatively largest diameter and a relatively smallest height, a second external fence 328-2 having a smaller diameter than the first external fence 328-1 and a larger height than the first external fence 328-1, and a third external fence 328-3 having a smaller diameter than the second external fence 328-2 and a larger height than the second external fence 328-2. A middle frame 330 and the operation part 500, which will be described below, are disposed in an internal space formed by the third external fence 328-3. A skirt 372 of the top case 370 to be described below is seated on an upper surface of the second external fence 328-2. When the top case 370 is seated on the upper surface of the second external fence 328-2, the third external fence 328-3 is covered by the top case 370 and is not exposed to the outside. The top case restriction member 380 to be described below is seated on an upper surface of the first external fence 328-1. A coupling groove 328-1R is formed in the upper surface of the first external fence 328-1, and a first coupling protrusion 381 of the top case restriction member 380 is inserted into the coupling groove 328-1R. A coupling groove 328-2R is formed in the lower portion of the second external fence 328-2, and a second coupling protrusion 382 of the top case restriction member 380 is inserted into the coupling groove 328-2R. The coupling protrusions 381 and 382 of the top case restriction member 380 may be coupled to the coupling grooves 328-1R and 328-2R of the base frame 310 to fix the top case restriction member 380 to the base frame 310. As a result, the top case 370 seated on the upper surface of the second external fence 328-2 of the base frame 310 may be restricted by the base frame 310.

The middle frame 330 is disposed on the base frame 310 to support the insertion unit assembly 400 to be described below. The insertion unit assembly 400 is inserted into the middle frame 330. The middle frame 330 includes a stage 331 that supports the operation part 500 to be described below. A middle frame opening 332 connected to the base frame opening 314 of the base frame 310 may be formed in a central portion of the stage 331. At least a part of the insertion unit assembly 400 to be described below may be inserted into the base frame opening 314 through the middle frame opening 332.

Guide protrusions 334 may be disposed on the middle frame 330. Specifically, the guide protrusions 334 may be disposed at both sides of the stage 331 of the middle frame 330. The guide protrusion 334 may guide the operation part 500 to move in a third direction (see a direction illustrated in FIGS. 19, 20, and 23 to 27) perpendicular to the first direction (see the direction illustrated in FIGS. 19, 20, and 23 to 27). That is, the guide protrusion 334 may guide the operation part 500 to move from a deactivation position to an activation position. Here, the deactivation position may be a position at which the operation part 500 does not allow the sensor unit 100 and the insertion unit 430 to move in the first direction (see the direction illustrated in FIGS. 19, 20, and 23 to 27). The activation position may be a position at which the operation part 500 allows the sensor unit 100 and the insertion unit 430 to move in the first direction (see the direction illustrated in FIGS. 19, 20, and 23 to 27).

A locking unit may be formed on the middle frame 330. The locking unit can prevent the operation part 500 from moving in the third direction, that is, the operation part 500 from moving from the deactivation position to the activation position. The locking unit may be disposed in the through hole 336 formed in the stage 331. The locking unit includes a locking body 339 connected to the middle frame 330 to be engaged with or disengaged from the operation part 500. The locking body 339 includes a body portion 340 connected to the middle frame 330 to be elastically deformed, a hook 341 connected to one side of the body portion 340 to be engaged with the operation part 500, and a leg 342 connected to the other side of the body portion 340 to be in contact with the moving tab 600. When the leg 342 is in contact with the moving tab 600, the hook 341 may be biased in a direction to be engaged with the operation part 500. When the leg 342 is not in contact with the moving tab 600, the hook 341 may be biased in a direction to be disengaged from the operation part 500.

An elastic pressing part 345 may be disposed on the middle frame 330. The elastic pressing part 345 may elastically support the operation part 500. The elastic pressing part 345 may be in contact with the operation part 500 to elastically support the operation part 500 to prevent the operation part 500 from easily moving from the deactivation position to the activation position. That is, the elastic pressing part 345 generates a resistance force against a user's force pushing the operation part 500. Accordingly, the elastic pressing part 345 may be formed so that the operation part 500 moves in the third direction only when the user applies a force of a predetermined level or more to the operation part 500.

The middle frame 330 further includes a button guide 346 extending upward from the stage 331. The button guides 346 may guide a movement of a button part 510. A guide hole 347 is formed in the button guide 346. When the operation part 500 to be described below is coupled to the middle frame 330, a button of the button part 510 is disposed in the guide hole 347 of the middle frame 330. Meanwhile, when the top case 370 is fixed to the base frame 310 using the top case restriction member 380 to be described below, at least a part of the button guide 346 of the middle frame 330 is exposed to the outside through an top case opening 371 of the top case 370 along with the button part 510 of the operation part 500.

The locking hook 360 may be coupled to the base frame 310. The locking hook 360 may be engaged with the base unit 200 accommodated in the recess 313 to maintain the coupled state of the base unit 200 and the base frame 310. The locking hook 360 may be disengaged from the base unit 200 after the sensing device 20 is attached to the user's skin.

The locking hook 360 may be pivot-coupled to the support 318 of the base frame 310. The locking hook 360 includes a pivot portion 361 rotatably coupled to the support 318, a hook portion 362 protruding from one side of the pivot portion 361, an extension 363 protruding from the other side of the pivot portion 361, and an expansion 364 protruding from an end portion of the pivot portion 361.

The pivot portion 361 is rotatably disposed between the elastic support 318-1 and the fence part 316. The support protrusion 318-2 of the support 318 is in contact with the pivot portion 361 at an upper side of the pivot portion 361 to prevent the pivot portion 361 from being separated between the elastic support 318-1 and the fence part 316. In addition, the pivot portion 361 may slide in a longitudinal direction thereof while supported by the support 318.

The hook portion 362 may protrude from one side of the pivot portion 361 and have an end portion inserted into the housing groove 217 of the base unit 200. When the end portion of the hook portion 362 is inserted into the housing groove 217 of the base unit 200, the movement of the extension 363 is restricted by the operation part 500. Accordingly, the movement of the locking hook 360 may be restricted by the operation part 500 to maintain the coupled state of the base unit 200 and the base frame 310, that is, the coupled state of the base unit 200 and the applicator body 300. Meanwhile, when the sensor unit 100 is moved in the first direction and coupled to the base unit 200, the restriction of the extension 363 by the operation part 500 is released. Accordingly, the end portion of the hook portion 362 may be separated from the housing groove 217 of the base unit 200.

The expansion 364 protrudes from an end portion of the pivot portion 361. The expansion 364 is disposed at each of both end portions of the pivot portion 361. The expansion 364 has a larger diameter than the pivot portion 361. The expansion 364 may be in contact with the elastic support 318-1 to restrict the movement of the pivot portion 361 and limit a moving range of the pivot portion 361 in the longitudinal direction.

The locking hook 360 may be moved in the third direction by a stopper protrusion 535 of the operation part 500. The locking hook 360 moved in the third direction may not move in a fourth direction due to the retention protrusion 327.

Specifically, the stopper protrusion 535 of a stopper 520 will be described below may be in contact with the locking hook 360 only when the operation part 500 moves in the third direction and may be not in contact with the locking hook 360 when the operation part 500 that has moved in the third direction moves in the fourth direction. The stopper protrusion 535 includes a stopper protrusion contact portion 536 and a stopper protrusion detent 537. The stopper protrusion contact portion 536 may be in contact with an upper surface of the extension 363 of the locking hook 360 to restrict a tilting motion of the locking hook 360. The stopper protrusion detent 537 may be in contact with one side portion of the extension 363 to push the locking hook 360 and may release a fixed mode of the locking hook 360.

As illustrated in FIGS. 14A and 21A, when the operation part 500 is positioned at the deactivation position, the stopper protrusion contact portion 536 is in contact with the locking hook 360 to restrict the tilting motion of the locking hook 360. In this case, the locking hook 360 may be engaged with the base unit 200 to maintain a coupling relationship between the base unit 200 and the applicator body 300. In this case, as illustrated in FIG. 14A, the retention protrusion 327 may be in contact with a side portion of the locking hook 360.

As illustrated in FIGS. 14B and 21B, when the operation part 500 is moved in the third direction by the user's manipulation, the stopper protrusion detent 537 of the stopper protrusion 535 moves the locking hook 360 in the third direction. In this case, the expansion 364 of the locking hook 360 may pass through the retention protrusion 327 while elastically deforming the retention protrusion 327. When the expansion 364 completely passes through the retention protrusion 327, the retention protrusion 327 may return to its original state and may be in contact with one end portion of the locking hook 360.

Referring to FIG. 14C, when the user's pressure on the operation part 500 in the third direction is removed, the stopper protrusion 535 is disengaged from the locking hook 360. Specifically, when the user's pressure on the operation part 500 in the third direction is removed, the operation part 500 moves in the fourth direction, but the locking hook 360 may not move in the fourth direction due to the retention protrusion 327. Accordingly, the stopper protrusion 535 is detached from the locking hook 360. In the present embodiment, the operation part 500 may be moved in the fourth direction to return to an initial deactivation position. As illustrated in FIG. 14C, when the operation part 500 is positioned at the initial deactivation position, the stopper protrusion 535 is disengaged from the locking hook 360. Accordingly, the locking hook 360 may tilt, and the base unit 200 may be detached from the applicator body 300.

The top case 370 is coupled to the main bodies 310 and 330 to cover the insertion unit assembly 400 and the operation part 500, which will be described below. Specifically, the top case 370 is seated on the base frame 310 and fixed to the base frame 310 through the top case restriction member 380 to be described below. Accordingly, the top case 370 may cover at least a part of the base frame 310, the middle frame 330, the insertion unit assembly 400, and the operation part 500. The top case 370 has the top case opening 371 that exposes the button part 510 of the operation part 500, which will be described below, to the outside. The skirt 372 extending in an outer diameter direction is formed on a lower end portion of the top case 370. The skirt 372 is seated on an upper surface of the second outer fence 328-2 of the base frame 310.

At least a part of the applicator body 300 is formed of a molded pulp. The molded pulp may be manufactured by injecting a pulp solution, which decomposes water and waste paper or a pure pulp, into a mold, vacuum suction forming, and then drying. The molded pulp may replace a synthetic resin that is used as a material for the conventional applicator body, thereby minimizing environmental destruction. For example, at least one of the base frame 310, the middle frame 330, and the top case 370 of the applicator body 300 may be formed of a molded pulp. Hereinafter, for convenience of description, it is assumed that at least a part of the top case 370 is formed of a molded pulp, but the scope of the present invention is not limited thereto.

At least a part of the top case 370 is formed of a molded pulp. For example, at least some regions of the top case 370 may be formed of a molded pulp and the remaining region may be formed of a material other than a molded pulp. Here, some regions and the remaining region may be coupled by physical, mechanical, and chemical methods. As another example, the top case 370 may be a single piece formed of a molded pulp. In the former case, each region of the top case 370 may be formed of a different material. In the latter case, the top case 370 may be formed by a relatively simple method. Any component for coupling the top case 370 to the base frame 310 may not be formed on an inner surface of the top case 370.

The molded pulp may include a pulp fiber and a filler.

The pulp fiber may include one or more fibers selected from the group consisting of a wood pulp fiber and a non-wood pulp fiber depending on a raw material from which fibers are obtained. The wood pulp fiber may include a fiber obtained from a softwood pulp (SwP), a hardwood pulp (HwP), or a mixture thereof. The non-wood pulp fiber may include a fiber obtained from a straw pulp, a bagasse pulp, a reed pulp, a bamboo pulp, a bast fiber pulp, a rag pulp, a cotton pulp, or a mixture thereof. The pulp fiber may be formed of a pulp produced by mechanically and/or chemically processing wood or other fibrous plant bodies depending on a processing method of the raw material. For example, the pulp fiber may include a fiber obtained from mechanical pulps such as a ground wood pulp (GP), a refiner pulp (RP), and a thermomechanical pulp (TMP) obtained by mechanically crushing wood or other plant bodies; chemical pulps such as a kraft pulp (KP), a sulphite pulp (SP), and a semi-chemical pulp (SCP) obtained by treating wood or other plant bodies with various chemicals to remove non-fibrous substances; and waste paper pulps such as a waste paper pulp formed of waste paper and a deinking pulp (DIP); or a mixture thereof.

As a non-limiting example, the filler may include at least one of a reinforcing material for reinforcing the rigidity of a molded pulp, a moisture resistant material for improving moisture resistant properties of a molded pulp, and a fire resistant material for improving the thermal stability of a molded pulp. The filler may be, for example, in the form of short fibers or long fibers, or in the form of particles having at least one of a spherical shape and a needle shape, but the scope of the present invention is not limited thereto. In this case, the short fiber may be a fiber having a length of 500 µm to 3 mm but is not limited thereto. In addition, the short fiber may have a thickness of 0.5 to 10 denier per fiber length, for example, 0.5 to 6 denier, but is not limited thereto. In addition, the powder may have a particle size (D50) of 3 µm to 3 mm, but is not limited thereto. The filler may be, for example, dispersed between pulp fibers and distinguished from the pulp fiber. Alternatively, the filler may be integrated with the pulp fiber and may not be distinguished from the pulp fiber. Alternatively, the filler may be provided in the form of fiber to form a composite fiber structure along with the pulp fiber. The filler may include, for example, a petroleum-based polymer and/or a biodegradable polymer. The petroleum-based polymer may be a polyolefin-based polymer such as polyethylene terephthalate (PET), high density polyethylene (HDPE), or the like, polyvinyl alcohol (PVA), a blend thereof, or a copolymer of each monomer. The biodegradable polymer may be a polymer that is decomposed into a low-molecular compound by microorganisms in nature and, for example, may be one or more selected from the group consisting of polylactic acid (PLA), thermoplastic starch (TPS), aliphatic polyester (AP), polycaprolactone (PCL), polyglycolic acid (PGA), polybutylene succinate (PBS), polybutylene adipate terephthalate (PBAT), polyhydroxy alkanoate (PHA), and a mixture thereof, but is not limited thereto. In addition, as the biodegradable polymer, a biodegradable bio-derived polymer may be used. The biodegradable bio-derived polymer may be, for example, at least one selected from the group consisting of bio-polyethylene (Bio-PE), bio-polyethylene terephthalate (Bio-PET), bio-trimethylene terephthalate (Bio-PTT), bio-polyamide (Bio-PA), bio-polypropylene (Bio-PP), and a mixture thereof, but is not limited thereto.

The top case restriction member 380 is coupled to the base frame 310 to restrict the top case 370 seated on the base frame 310 to the base frame 310. The top case restriction member 380 may be formed in a ring shape that fully surrounds an outer circumference of a lower portion of the top case 370. The top case restriction member 380 is seated on the upper surface of the first outer fence 328-1 of the base frame 310 and coupled to the base frame 310. The coupling protrusions 381 and 382 to be coupled to the base frame 310 are formed on the top case restriction member 380. The first coupling protrusion 381 protrudes from a lower surface of the top case restriction member 380 facing the upper surface of the first external fence 328-1. The first coupling protrusion 381 is inserted into the coupling groove 328-1R formed in the upper surface of the first external fence 328-1. The second coupling protrusion 382 protrudes from an inner surface of the top case restriction member 380 facing an outer surface of the second external fence 328-2. The second coupling protrusion 382 is inserted into the coupling groove 328-2R formed in the outer surface of the second external fence 328-2. Meanwhile, a pressing protrusion 383 may be formed on an upper region of the inner surface of the top case restriction member 380. When the top case restriction member 380 is coupled to the base frame 310, the pressing protrusion 383 may press the top case 370 seated on the upper surface of the second external fence 328-2 of the base frame 310. Meanwhile, the top case restriction member 380 may have an upper inner diameter smaller than a lower inner diameter, but the scope of the present invention is not limited thereto.

The insertion unit assembly 400 moves the needle 485 in each of the first direction and the second direction that is a direction opposite to the first direction. Since the needle 485 moves in the first direction while coupled to the sensor unit 100, the insertion unit assembly 400 moves the sensor unit 100, which is one component of the sensing device 20, in the first direction. The insertion unit assembly 400 includes a column member 410 and an insertion unit 430. The insertion unit 430 includes a shuttle 431, a shuttle driver 450, a needle assembly 460, and a needle release driver 490.

The column member 410 is fixed to the applicator body 300 to support the insertion unit 430. The column member 410 includes a column member body 411 of which one end portion is formed with an outward open column member opening 412. A space for accommodating the insertion unit 430 is provided in the column member body 411. A release portion 414 is disposed on an inner surface of the column member body 411. The release portion 414 may come into contact with a carrier 462 while the carrier 462 of the needle assembly 460 to be described below moves in the first direction. As the carrier 462 is in contact with the release portion 414, the carrier 462 may be disengaged from the shuttle 431 to move relatively with respect to the shuttle 431.

A clamping portion 416 and a blocking portion 420 are disposed at both sides of the column member body 411 to restrict the movement of the shuttle 431. The clamping portion 416 has a clamping portion detent 417 that may be engaged with one side of the shuttle 431. The insertion unit assembly 400 may be coupled to the applicator body 300 in a state in which the shuttle 431 is temporarily fixed to the column member 410 by the clamping portion 416. When the clamping portion detent 417 of the clamping portion 416 is engaged with a shuttle latch 438 of the shuttle 431, the shuttle 431 is temporarily fixed to the column member 410. When the clamping portion 416 is used to temporarily fix the shuttle 431 to the column member 410, the problem of the shuttle 431 accidentally moving during the assembly process of the insertion unit assembly 400 may be prevented. During the manufacturing process of the applicator assembly 10, after the insertion unit assembly 400 is coupled to the applicator body 300, the clamping portion 416 may be disengaged from the shuttle 431. As illustrated in FIG. 9, the applicator assembly 10 may be provided to the user in a state in which the clamping portion 416 is disengaged from the shuttle 431. The blocking portion 420 may be used to fix the shuttle 431 that has moved in the first direction to the corresponding position. The blocking portion 420 has a blocking portion detent 421 engaged with one side of the shuttle 431. The blocking portion 420 can prevent the shuttle 431 from moving in the second direction, which is a direction opposite to the first direction, by engaging the blocking portion detent 421 with a detent portion 439 of the shuttle 431 after the shuttle 431 has moved in the first direction. The blocking portion 420 can prevent the shuttle 431 and the applicator 30 from being reused by restricting the movement of the shuttle 431.

The shuttle 431 is disposed inside the column member 410. The shuttle 431 is disposed to linearly move with respect to the column member 410. The shuttle 431 includes a shuttle body 432. A space that accommodates the needle assembly 460 to be described below is provided inside the shuttle body 432. A shuttle protrusion 434 is disposed at one side of the shuttle body 432. The shuttle protrusion 434 protrudes from the shuttle body 432 to be in contact with the operation part 500. The shuttle protrusion 434 prevents or allows the movement of the shuttle 431 in the first direction along with the operation part 500 to be described below. Specifically, when the shuttle protrusion 434 is in contact with the operation part 500, the movement of the shuttle 431 in the first direction is prevented. When the shuttle protrusion 434 is not in contact with the operation part 500, the movement of the shuttle 431 in the first direction is allowed. A shuttle detent 436 that may be engaged with a carrier latch 476 of the carrier 462, which will be described below, is formed inside the shuttle body 432. When the shuttle detent 436 is engaged with the carrier latch 476 to be described below, the carrier 462 may not move relatively with respect to the shuttle 431. When the shuttle detent 436 is disengaged from the carrier latch 476, the carrier 462 may move relatively with respect to the shuttle 431. A shuttle through hole 441 is formed in a shuttle bottom portion 440 of the shuttle body 432. The needle 485 may protrude outward from the shuttle body 432 through the shuttle through hole 441. The push protrusion 442 is formed on the shuttle bottom portion 440. The push protrusion 442 may be inserted into the sensor unit groove 138 of the sensor unit 100. As the push protrusion 442 may be inserted into the sensor unit groove 138 of the sensor unit 100, the sensor unit 100 may maintain a right position without moving. A fixing part 444 is formed inside the shuttle 431. The fixing part 444 is coupled to the needle release driver 490.

The shuttle driver 450 is disposed between the column member 410 and the shuttle 431. The shuttle driver 450 may provide a moving force for the shuttle 431 to move in the first direction. The shuttle driver 450 includes a forward movement elastic member 451 that provides an elastic force that causes the shuttle 431 to move in the first direction. One end of the forward movement elastic member 451 may be coupled to the column member 410, and the other end of the forward movement elastic member 451 may be coupled to the shuttle 431. The shuttle 431 may be installed inside the column member 410 in a state of elastically deforming the forward movement elastic member 451. In this case, the shuttle protrusion 434 is in contact with the operation part 500 to prevent the shuttle 431 from moving in the first direction. Then, when the operation part 500 moves in the third direction so that the shuttle protrusion 434 is not in contact with the operation part 500, the shuttle 431 is moved in the first direction by the elastic force of the forward movement elastic member 451. The forward movement elastic member 451 may be a coil spring, but the scope of the present invention is not limited thereto.

The needle assembly 460 may be coupled to the shuttle 431 so that at least a part of the needle assembly 460 may be disposed in the shuttle 431. The needle assembly 460 includes the carrier 462 and the needle 485.

The carrier 462 is coupled to the shuttle 431 to relatively move with respect to the shuttle 431. The carrier 462 may be partially engaged with the shuttle 431 to move in the first direction along with the shuttle 431. After moving in the first direction, the carrier 462 may be disengaged from the shuttle 431 and moved in the second direction. The carrier 462 includes a carrier body 463, at least one carrier wing 471, and at least one grip arm 478.

The carrier body 463 is coupled to the needle 485. The carrier body 463 is provided with a fixing part 469 to which the needle release driver 490 to be described below is coupled.

The carrier wing 471 extends from the carrier body 463. The carrier wing 471 may be at least partially elastically deformed when moving in the first direction. The carrier wing 471 includes a wing body 472 connected to the carrier body 463 to be elastically deformed, a trigger 474 protruding from the wing body 472, and the carrier latch 476. The trigger 474 may be in contact with the release portion 414 of the column member 410 while the carrier 462 and the shuttle 431 move together in the first direction. The carrier latch 476 protrudes outward from the wing body 472. The carrier latch 476 may be engaged with the shuttle detent 436 of the shuttle 431. In a state in which the carrier latch 476 is engaged with the shuttle detent 436, the carrier 462 may maintain the elastically deformed state of a backward movement elastic member 491 of the needle release driver 490. Accordingly, in a state in which the carrier latch 476 is engaged with the shuttle detent 436, the carrier 462 may not move in the second direction in which the elastic force of the backward movement elastic member 491 acts. As described above, when the trigger 474 is in contact with the release portion 414 and the carrier wing 471 is elastically deformed while the carrier 462 and the shuttle 431 move together in the first direction, the carrier latch 476 is separated from the shuttle detent 436.

The grip arm 478 is provided on a side portion of the carrier body 463 to be elastically deformed. The grip arm 478 may be disposed so that a plurality of grip arms 478 face each other at both sides of the carrier body 463 to be engaged with the sensor unit 100. The grip arm 478 has a grip protrusion 480 engaged with the sensor unit housing 120 and a protrusion 482 in contact with an inner wall 424 of the column member 410. The carrier 462 is coupled to the shuttle 431 so that the carrier body 463 is positioned inside the shuttle 431 and an end portion of the grip arm 478 protrudes from the shuttle 431. The grip arm 478 may fix the sensor unit 100 in an engagement manner with the sensor unit housing 120. In this case, a surface of the sensor unit housing 120 is in close contact with the shuttle bottom portion 440 of the shuttle 431, and the push protrusion 442 of the shuttle 431 is inserted into the sensor unit groove 138 of the sensor unit housing 120.

As illustrated in FIG. 9, the carrier 462 may position the sensor unit 100 at a first position when the insertion unit assembly 400 is coupled to the applicator body 300. When the carrier 462 is positioned at the first position, the protrusion 482 of the grip arm 478 is in contact with the inner wall 424 of the column member 410. Accordingly, the grip arm 478 may be biased in a direction to be engaged with the sensor unit housing 120, and the coupled state of the carrier 462 and the sensor unit 100 may be maintained. Meanwhile, when the sensor unit 100 and the carrier 462 move in the first direction and the sensor unit 100 is coupled to the base unit 200, the protrusion 482 is separated from the inner wall 424 of the column member 410. Accordingly, in a state in which the sensor unit 100 and the base unit 200 are coupled, the fixing strength of the grip arm 478 is weakened. Since the sensor unit 100 is coupled to the base unit 200 by the adhesive layer 230, when the carrier 462 is pulled in the second direction away from the sensing device 20 by the needle release driver 490, the grip arm 478 may be separated from the sensor unit 100.

The needle 485 is coupled to the carrier 462. The needle 485 may assist the sensor 110 when at least a part of the sensor 110 is inserted into the human body. To this end, the needle 485 may be moved from the first position in the first direction along with the sensor unit 100 in a state of being coupled to the sensor unit 100 and inserted into the user's skin. In addition, the needle 485 may be moved from a second position in the second direction along with the carrier 462 after moving in the first direction and detached from the sensor unit 100. The needle 485 may have a sharp end to pierce the user's skin and be smoothly inserted into the user's skin. The needle 485 pierces the user's skin before the sensor 110 when the sensor unit 100 moves in the first direction so that the sensor 110 is stably inserted into the skin. The needle 485 includes a needle body 486 that may be inserted into the user's skin, and a needle head 487 coupled to the carrier 462. A needle coupler 489 for fixing the needle head 487 to the carrier 462 is coupled to the needle head 487. The needle coupler 489 may be fixed to an inner side of the carrier 462 to fix the needle head 487 to the carrier 462.

The needle release driver 490 is disposed between the shuttle 431 and the needle 485 and moves the needle 485 in the second direction. Specifically, one end of the needle release driver 490 is coupled to the shuttle 431, and the other end of the needle release driver 490 is coupled to the carrier 462 to which the needle 485 is coupled. The needle release driver 490 may provide a driving force for the carrier 462 to move in the second direction. The carrier 462 may be moved relatively with respect to the shuttle 431 by the needle release driver 490. The needle release driver 490 includes the spring-shaped backward movement elastic member 491 in a tensioned state. The backward movement elastic member 491 applies an elastic force to the carrier 462 in a direction away from the sensor unit 100. That is, the backward movement elastic member 491 may move the carrier 462 and the needle 485 so that the carrier 462 and the needle 485 are detached from the sensor unit 100. One end of the backward movement elastic member 491 is coupled to the fixing part 444 of the shuttle 431. During the assembly process of the insertion unit 430, one end of the backward movement elastic member 491 enters a fixing part groove 445 through an inlet 448. One end of the backward movement elastic member 491 is in close contact with a contact surface 446 by the elastic force of the backward movement elastic member 491 in a state of entering the fixing part groove 445. In this case, one end of the backward movement elastic member 491 is blocked by a barrier part 447 and thus cannot be separated from the fixing part 444 by the method of passing through the inlet 448. Accordingly, the backward movement elastic member 491 may maintain a state of being stably coupled to the fixing part 444. The other end of the backward movement elastic member 491 is fixed to the fixing part 469 of the carrier 462. The fixing part 469 of the carrier 462 may also be formed of the configuration that is the same as or different from that of the fixing part 444 of the shuttle 431. The backward movement elastic member 491 may be a coil spring, but the scope of the present invention is not limited thereto.

The insertion unit assembly 400 may be coupled in the applicator body 300 in a state in which the forward movement elastic member 451 of the shuttle driver 450 that moves the shuttle 431 and the backward movement elastic member 491 of the needle release driver 490 that moves the needle assembly 460 are elastically deformed. In addition, the insertion unit assembly 400 may be disposed in the applicator body 300 in a state of being coupled to the sensor unit 100. According to one embodiment of the present invention, a specific configuration of the insertion unit 430 may be simply assembled to the applicator body 300 by form a single assembly along with the column member 410.

The operation part 500 is disposed in the applicator body 300. The operation part 500 is coupled to the insertion unit assembly 400 in the applicator body 300. The operation part 500 is coupled to the applicator body 300 so that the movement from the deactivation position to the activation position (the movement in the third direction) is possible by the user's manipulation. The movement of the operation part 500 in the third direction allows the movement of the sensor unit 100, the shuttle 431, and the needle assembly 460 in the first direction. The operation part 500 includes the button part 510, the stopper 520, and the stopper protrusion 535.

The button part 510 is disposed on the button guide 346 of the middle frame 330. The end portion of the button part 510 may be exposed to the top case opening 371 of the applicator body 300 through the guide hole 347 of the middle frame 330 and pressed in the third direction by the user. When the user presses the button part 510 exposed to the outside of the top case 370, the stopper 520 may also be pressed. The button part 510 may be elongated from one side portion of an upper surface of a stopper body 521. The button part 510 may include an elongation member elongated from the upper surface of the stopper body 521, and the button outwardly protruded from an upper portion of the elongation member.

The stopper 520 is disposed on the middle frame 330. The stopper 520 prevents and allows the movement of the sensor unit 100, the shuttle 431, and the needle assembly 460 in the first direction. The stopper 520 includes a stopper body 521 and a support 526. The button part 510 is formed to extend from an upper surface of the stopper body 521 on one end portion of the upper surface of the stopper body 521. As a non-limiting example, the operation part 500 may be formed by at least integrating the button part 510 and the stopper body 521. As a non-limiting example, the button part 510, the stopper 520, and the stopper protrusion 535 may be integrally formed to form the operation part 500.

The stopper body 521 forms an overall exterior of the stopper 520. The stopper body 521 is supported by the stage 331 of the middle frame 330. A stopper opening 522 is formed in a central portion of the stopper body 521. The insertion unit assembly 400 including the column member 410 is inserted into the stopper opening 522.

A stopper detent 524 is formed at one side of the stopper body 521. The stopper 520 may not move in the third direction in a state in which the hook 341 of the locking unit 338 of the middle frame 330 is engaged with the stopper detent 524. That is, in this case, even when the button part 510 is manipulated, the stopper 520 may not move from the deactivation position to the activation position. The stopper 520 may move to the activation position in a state in which the locking body 339 is disengaged from the stopper detent 524.

The support 526 is disposed to protrude from the stopper body 521 around the stopper opening 522. The support 526 may support the shuttle protrusion 434 of the shuttle 431. When the stopper 520 is positioned at the deactivation position, the support 526 may support the shuttle protrusion 434 to prevent the shuttle 431 from moving in the first direction. When the stopper 520 is moved in the third direction, that is, when the stopper 520 is positioned at the activation position, the support 526 does not support the shuttle protrusion 434 to allow the shuttle 431 to move in the first direction.

The stopper protrusion 535 is disposed on a lower surface of the stopper body 521. The stopper protrusion 535 protrudes from the stopper body 521 to be in contact with one side of the locking hook 360. When the stopper 520 is positioned at the deactivation position, the stopper protrusion 535 restricts the movement of the locking hook 360 to prevent the locking hook 360 from tilting. When the stopper 520 moved to the activation position is moved to deactivation position from the activation position, the stopper protrusion 535 releases the restriction of the locking hook 360. Specifically, the stopper protrusion 535 includes the stopper protrusion contact portion 536 and the stopper protrusion detent 537. The stopper protrusion contact portion 536 may be in contact with the upper surface of the extension 363 of the locking hook 360 to restrict the tilting motion of the locking hook 360. The stopper protrusion detent 537 may be in contact with one side portion of the extension 363 to push the locking hook 360 and may release the fixed mode of the locking hook 360.

When the stopper 520 moves from the deactivation position illustrated in FIGS. 19 and 21A to the activation position illustrated in FIGS. 20 and 21B, the stopper protrusion 535 is positioned above the extension 363 of the locking hook 360. **In** this case, the stopper protrusion detent 537 of the stopper protrusion 535 moves the locking hook 360 in the third direction, and during the corresponding process, the stopper protrusion contact portion 536 of the stopper protrusion 535 is in contact with an upper surface of the extension 363 of the locking hook 360 to restrict the tilting motion of the locking hook 360. Accordingly, the locking hook 360 may not be tilted to be disengaged from the base unit 200. Meanwhile, after the processes illustrated in FIGS. 20 and 21B, the operation part 500 moves in the fourth direction, but the movement of the locking hook 360 in the fourth direction is restricted by the retention protrusion 327. Accordingly, the upper surface of the extension 363 of the locking hook is released from the restriction of the stopper protrusion contact portion 536. As a result, the locking hook 360 may tilt without interfering with the stopper protrusion 535.

The operation prevention part 40 is coupled to the applicator 30 to restrict the operation of the operation part 500. That is, the operation prevention part 40 prevents the operation part 500 from being moved in the third direction by an unintended behavior of the user (prevents the movement from the deactivation position to the activation position). In addition, the operation prevention part 40 moves along with the protective sheet 240 when removed from the applicator 30 to detach the protective sheet 240 from the adhesive layer 230. The operation prevention part 40 includes the moving tab 600.

At least a part of the moving tab 600 may be drawn out from the inside to the outside of the applicator body 300 through the doorway 322 of the applicator body 300. The moving tab 600 may be supported by the stage 320 of the applicator body 300 to move in the fourth direction.

The moving tab 600 restricts the movement of the operation part 500 including the stopper 520. As illustrated in FIG. 9, when the operation part 500 is positioned at the deactivation position and the moving tab 600 is positioned inside the applicator body 300, the moving tab 600 is in contact with the leg 342 of the locking unit 338. In this case, the hook 341 of the locking unit 338 is engaged with the stopper detent 524 of the operation part 500. In such a state, the stopper 520 is not allowed to move in the third direction. Meanwhile, when the moving tab 600 is drawn out to the doorway 322 and removed from the applicator body 300, the locking unit 338 may be disengaged from the operation part 500 so that the operation part 500 may move.

The moving tab 600 is coupled to the protective sheet 240. Accordingly, when the user moves the moving tab 600 in the fourth direction to remove the moving tab 600 from the applicator 30, the protective sheet 240 also moves in the fourth direction. As a result, the protective sheet 240 is detached from the adhesive layer 230. The moving tab 600 includes a moving tab body 610 and a holder 620.

The moving tab body 610 is connected to the protective sheet 240. An insertion hole 611 and a through hole 612 are formed in the moving tab body 610. The wing 242 of the protective sheet 240 may be inserted into the insertion hole 611. The through hole 612 may be formed to extend in a direction parallel to a moving direction of the holder 620. A handle part 614 is disposed on one end portion of the moving tab body 610, and a moving tab engagement part 616 is disposed on the other end portion of the moving tab body 610. When the moving tab 600 is positioned inside the applicator body 300, the handle part 614 is disposed outside the applicator body 300. A hook arm 617 engaged with the applicator body engagement part 324 of the base frame 310 is formed on the moving tab engagement part 616. When the moving tab 600 is positioned inside the applicator body 300, the hook arm 617 is engaged with the engagement protrusion 325. When a force of a predetermined level or more is applied to the moving tab 600 in the fourth direction, the hook arm 617 may be disengaged from the engagement protrusion 325. Accordingly, the moving tab 600 may be moved in the fourth direction.

The holder 620 is movably coupled to the moving tab body 610. The holder 620 includes a holding portion 621 and a pressing portion 622. In a state in which the wing 242 of the protective sheet 240 may be inserted into the insertion hole 611 of the moving tab body 610, the holding portion 621 may be inserted into the protective sheet hole 245 so that the protective sheet 240 may be coupled to the moving tab 600. The pressing portion 622 may protrude from the moving tab body 610 to press the protective sheet 240 in the first direction. That is, the pressing portion 622 presses the protective sheet 240 toward the adhesive layer 230 when the moving tab 600 is positioned inside the applicator body 300. Accordingly, the coupling strength between the protective sheet 240 and the adhesive layer 230 is prevented from weakening. Since the pressing portion 622 may be elastically deformed, when the moving tab 600 moves in the fourth direction, the pressing portion 622 may be elastically deformed by being in contact with the base frame 310.

The protective sheet 50 is coupled to the base unit 200. Specifically, the protective sheet 50 may be coupled to the base unit 200 in a form that covers the adhesive part 250 of the base unit 200. The protective sheet 50 may protect the adhesive part 250 until the user manipulates the applicator assembly 10 according to the present embodiment, thereby maintaining the adhesive strength of the adhesive part 250. The protective sheet 50 may include a releasable material, and the user can easily remove the protective sheet 50 from the adhesive part 250 of the base unit 200.

Hereinafter, a process of attaching the sensing device 20 to the user's skin using the applicator 30 according to the embodiment of the present invention will be described with reference to FIGS. 23 to 27.

First, as illustrated in FIG. 23, the protective sheet 240 is removed using the moving tab 600, which is the operation prevention part 40. When the moving tab 600 is moved in the fourth direction by the user, the moving tab 600 removes the protective sheet 240 from the adhesive layer 230 of the base unit 200. Meanwhile, when the moving tab 600 is moved in the fourth direction, the locking body 339 is free from the moving tab 600. In this case, the locking body 339 is disengaged from the operation part 500 so that the operation part 500 is switched to a state of being movable by the user.

Next, the protective sheet 50 is removed from the adhesive part 250 of the base unit 200 to expose the adhesive part 250, and the bottom portion 312 of the applicator body 300 and the adhesive part 250 are disposed in contact with the user's skin. Meanwhile, unlike the above description, the operation prevention part 40 may be removed after the protective sheet 50 is removed.

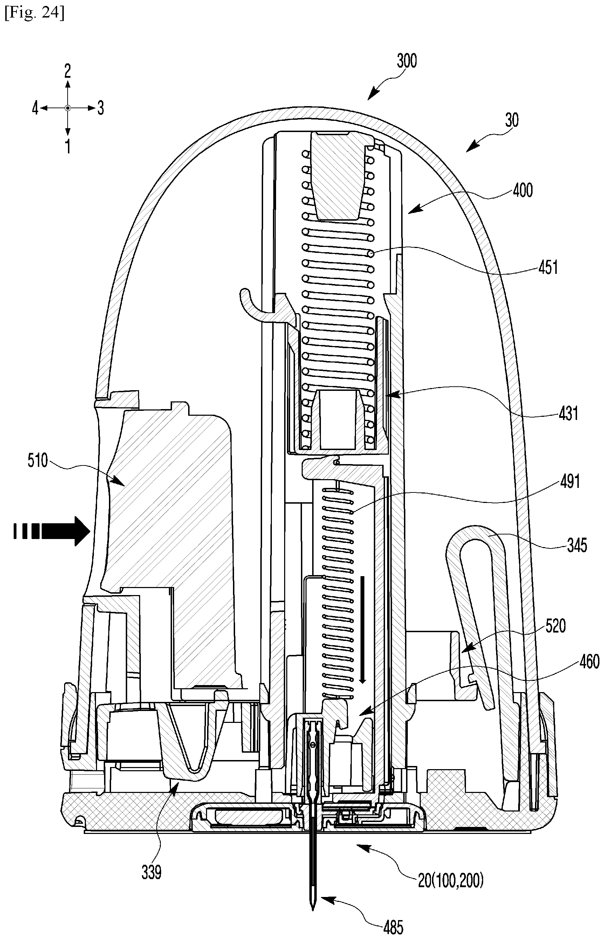

Next, as illustrated in FIG. 24, the operation part 500 is pressed so that the operation part 500 moves in the third direction. Accordingly, the sensor unit 100, the shuttle 431, and the needle assembly 460 are allowed to move in the first direction. Specifically, when the user presses the button part 510 of the operation part 500 in the third direction, the operation part 500 is moved in the third direction while elastically deforming the elastic pressing part 345 and is positioned at the activation position. As the operation part 500 moves to the activation position, the shuttle 431 is separated from the stopper 520 and moved in the first direction by the forward movement elastic member 451. As the shuttle 431 moves in the first direction, the needle 485 and the sensor 110 are inserted into the user's skin, and the sensor unit 100 is attached to the base unit 200 by the adhesive layer 230.

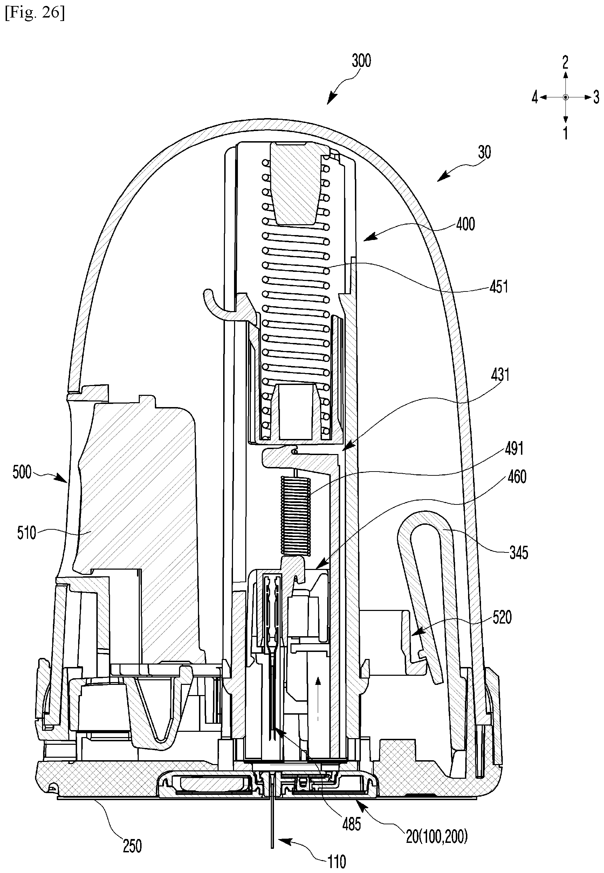

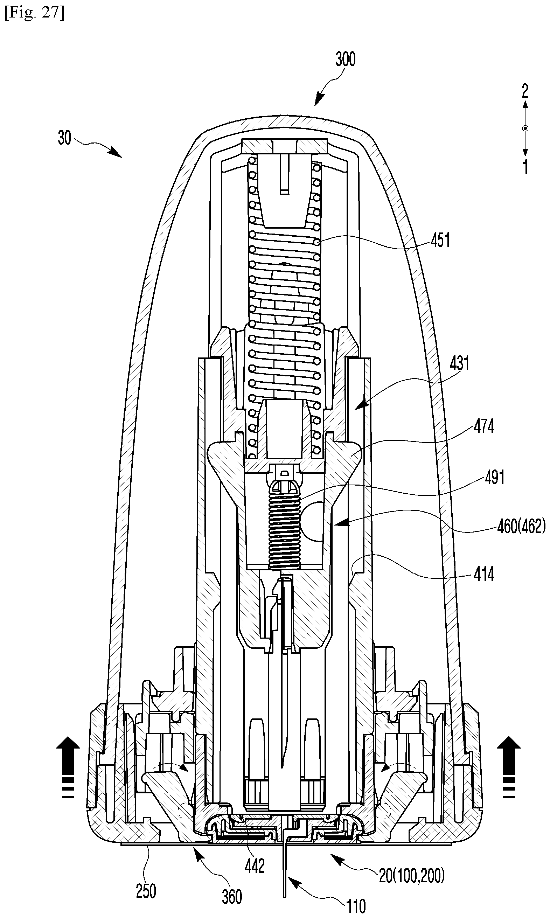

Meanwhile, when the shuttle 431 moves in the first direction, the trigger 474 of the carrier 462 comes into contact with the release portion 414 of the column member 410 so that the carrier latch 476 is disengaged from the shuttle detent 436. When the carrier 462 is disengaged from the shuttle 431, as illustrated in FIGS. 26 and 27, the carrier 462 is moved in the second direction away from the user's skin by the backward movement elastic member 491. In this case, the needle 485 is separated from the user's skin and the sensing device 20 maintains a state of being attached to the user's skin by the adhesive part 250. While the needle 485 moves in the second direction, the shuttle bottom portion 440 of the shuttle 431 moved in the first direction maintains a state of being in contact with the sensor unit 100. In addition, the push protrusion 442 of the shuttle 431 may maintain a state of being inserted into the sensor unit groove 138 of the sensor unit 100.

After the sensor 110 is inserted into the user's skin, the applicator 30 may be separated from the sensing device 20 attached to the skin. Specifically, when the pressing applied to the operation part 500 in the third direction is released, the operation part 500 moves in the fourth direction, but the locking hook 360 cannot move in the fourth direction, and as a result, the extension 363 of the locking hook 360 is free from the stopper protrusion 535. Accordingly, the locking hook 360 becomes a tiltable state. Accordingly, as illustrated in FIG. 27, when the user lifts the applicator 30 from the sensing device 20, the locking hook 360 tilts so that the applicator 30 may be disengaged from the sensing device 20.

Although embodiments of the present invention have been described above, those skilled in the art may modify and change the present invention in various ways by the addition, change, or omission of components without departing from the spirit of the present invention stated in the appended claims, which will also be included in the scope of the present invention.

### [Explanation of the Signs]

10: applicator assembly
20: sensing device
30: applicator
40: operation prevention part
50: protective sheet
100: sensor unit
200: base unit
300: applicator body
400: insertion unit assembly
500: operation part
600: moving tab

## Claims

1. An applicator assembly comprising:
a sensing device; and
an applicator detachably coupled to the sensing device,
wherein the applicator includes:
an applicator body covering the sensing device and of which at least a part is formed of a molded pulp;
an insertion unit assembly including a needle and disposed in the applicator body, the insertion unit assembly is configured to move at least a part of the sensing device and the needle in a first direction and is configured to move the needle in a second direction opposing the first direction; and
an operation part disposed in the applicator body, coupled to the insertion unit assembly, and configured to allow the at least a part of the sensing device and the needle to move in the first direction, and
the operation part includes:
a button part which is movable in a third direction perpendicular to the first direction and of which at least a part is exposed to the outside of the applicator body; and
a stopper which extends from the button part, prevents the insertion unit assembly from moving in the first direction, and allows the insertion unit assembly to move in the first direction by movement of the button part in the third direction.

2. The applicator assembly of claim 1, wherein the button part and the stopper are formed integrally.

3. The applicator assembly of claim 1, wherein the applicator body includes:
a base frame having a recess formed on a lower surface of the base frame; and
a top case seated on the base frame to cover the base frame and operation part and of which at least a part is formed of a molded pulp, and
the at least a part of the button part is exposed to the outside through an opening formed in the top case.

4. The applicator assembly of claim 3, wherein the top case is a single piece of the molded pulp.

5. The applicator assembly of claim 3 or claim 4, wherein the applicator body further includes a middle frame which is disposed on the base frame and supports the insertion unit assembly by inserting the insertion unit assembly thereinto.

6. The applicator assembly of claim 5, wherein the middle frame includes:
a stage which is disposed on the base frame and supports the insertion unit assembly by inserting the insertion unit assembly thereinto; and
a button guide in which a guide hole is formed and which extends upward from the stage to guide the button part.

7. The applicator assembly of claim 6, wherein the at least a part of the button part is disposed in the guide hole of the button guide.

8. The applicator assembly of claim 7, wherein the insertion unit assembly further includes:
a column member disposed in a middle frame opening formed in the stage;
a shuttle which is disposed in the column member and in which the needle is coupled thereto;
a shuttle driver which is disposed between the column member and the shuttle and moves the shuttle in the first direction; and
a needle release driver which is disposed between the shuttle and the needle and moves the needle in the second direction.

9. The applicator assembly of claim 8, wherein the stopper includes:
a stopper body in which an opening into which the column member is inserted is formed and which is movable in the third direction by the button part; and
a support which is disposed on at least a part around the opening of the stopper, supports the shuttle, and allows the shuttle to move in the first direction by movement of the stopper body in the third direction.

10. The applicator assembly of claim 9, wherein the insertion unit assembly further includes a carrier disposed between the needle release driver and the needle, and
the carrier is engaged with the shuttle to move in the first direction along with the shuttle, and when the shuttle completely moves in the first direction, the carrier is disengaged from the shuttle to move in the second direction.

11. The applicator assembly of claim 1, wherein the molded pulp includes a pulp fiber and a filler.

12. The applicator assembly of claim 11, wherein the filler includes a moisture resistant material.

13. The applicator assembly of claim 10, further comprising an operation prevention part which is coupled to the applicator and restricts the operation part.

14. The applicator assembly of any one of claim 1 to claim 13, wherein the sensing device includes:
a sensor unit coupled to the insertion unit assembly to move in the first direction and including a sensor configured to sense biometric data; and
a base unit disposed in the recess of the base frame and spaced apart from the sensor unit.

15. The applicator assembly of any one of claim 1 to claim 14, wherein the operation part moves in a third direction perpendicular to the first direction to allow the sensor unit to move in the first direction, and
the operation prevention part is inserted into the base frame to restrict the movement of the operation part in the third direction.
